(19) Europäisches Patentamt
European Patent Office
Office européen des brevets

(11) **EP 2 223 648 A1**

(12) **EUROPEAN PATENT APPLICATION**

(43) Date of publication:
**01.09.2010 Bulletin 2010/35**

(51) Int Cl.:
**A61B 5/00** (2006.01)

(21) Application number: **10153553.2**

(22) Date of filing: **03.02.2010**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **17.02.2009 US 372662**

(62) Document number(s) of the earlier application(s) in accordance with Art. 76 EPC:
**10152573.1 / 2 218 394**

(71) Applicants:
• **Bardy, Gust H.**
  **Redmond WA 98053 (US)**
• **Alferness, Clifton A.**
  **Port Orchard, WA 98367-7450 (US)**

(72) Inventors:
• **Bardy, Gust H.**
  **Redmond WA 98053 (US)**
• **Alferness, Clifton A.**
  **Port Orchard, WA 98367-7450 (US)**

(74) Representative: **Curley, Donnacha John et al**
  **Hanna Moore & Curley**
  **13 Lower Lad Lane**
  **Dublin 2 (IE)**

Remarks:
This application was filed on 14-02-2010 as a divisional application to the application mentioned under INID code 62.

(54) **System and method for providing a personalized tool for estimating glycated hemogloblin**

(57) A system (330) and method (280) for providing a personalized tool for estimating glycated hemoglobin is provided. An electronically-stored history of empirically measured glucose levels (240) for a patient (21) is maintained over a set period of time in order of increasing age. A decay factor (334) is applied to each of the measured glucose levels (240). The measured glucose levels (240) are aggregated and scaled as decayed into an estimate of glycated hemoglobin (337) for the time period. The glycated hemoglobin estimate (337) is displayed to the patient (21).

**Fig. 28.**

EP 2 223 648 A1

## Description

### Field

This application relates in general to diabetes mellitus management and, in particular, to a system and method for providing a personalized tool for estimating glycated hemoglobin.

### Background

Diabetes mellitus, or simply, "diabetes," is an incurable chronic disease. Type 1 diabetes is caused by the destruction of pancreatic beta cells in the Islets of Langerhans through autoimmune attack. Type 2 diabetes is due to defective insulin secretion, insulin resistance, or reduced insulin sensitivity. Gestational diabetes first appears during pregnancy and generally resolves after childbirth, absent preexisting weak pancreatic function. Less common forms of diabetes include thiazide-induced diabetes, and diabetes caused by chronic pancreatitis, tumors, hemochromatosis, steroids, Cushing's disease, and acromegaly.

Diabetes exacts a significant cost. In the United States, the annual healthcare costs of diabetes exceeds $200 billion. Additionally, the personal toll from diabetes is wide-ranging, having an impact on every patient's health and quality of life, as well as affecting the lives of the people around them. The unceasing demands of diabetes management can leave a patient feeling a loss of personal freedom, yet better control over diabetes lowers the risk of acute and chronic complications.

Type 1 diabetes can only be treated by taking insulin and making permanent lifestyle adjustments. Blood glucose and proper insulin dosing requires every Type 1 diabetic to play an active role in their own self-care. Whereas well-controlled Type 2 diabetics see relatively constrained rises and dips in blood glucose, Type 1 diabetics frequently experience wide fluctuations, known as lability or brittleness. Thus, the timing and dosing of insulin and patient-related factors, such as meals, exercise, and physiological condition, make effective blood glucose management a delicate balancing act between the prevention of hyperglycemia, or high blood glucose, and the frequent and serious consequences of hypoglycemia, or very low blood glucose, from over-aggressive or incorrect insulin dosing, which can lead to abrupt loss of consciousness.

In contrast, Type 2 diabetes is a progressive disease that requires increasing care as insulin resistance increases and insulin secretion diminishes. Initially, Type 2 diabetes can be managed through changes in physical activity, diet, and weight loss, which may temporarily restore normal insulin sensitivity. However, as insulin production becomes impaired, antidiabetic medications may be necessary to increase insulin production, decrease insulin resistance, and help regulate inappropriate hepatic glucose release. Eventually, insulin therapy will become necessary as insulin production ceases entirely.

Blood glucose management for both Type 1 and Type 2 diabetes is open loop. For a diabetic, well-controlled blood glucose falls between 70 mg/dL and 120 mg/dL before meals and under 140 mg/dL two hours after eating. Meal planning, insulin dosing, and physical activities are all effected by blood glucose. Patient-operable devices for automatically dosing insulin, based on real time blood glucose testing, are not yet available. Current blood glucose management instead requires regular self-testing using test strips and a blood glucose meter.

At-home self testing of blood glucose is generally supplemented with hospital testing of glycated hemoglobin (HbA1c), which measures the overall effectiveness of long-term blood glucose control. HbA1c reflects the tendency of glucose to bind to hemoglobin proteins over the 120-day lifespan of red blood cells. HbA1c is ordinarily determined by physicians in-laboratory through high performance liquid or Boronate affinity chromatography, or by immunoassay. HbA1c testing tends to weigh blood glucose levels over the most recent two weeks more heavily than the preceding ten weeks and consequently reflects a near term bias. Nevertheless, HbA1c remains the best measure of diabetes control.

Historically, physicians used HbA1c to identify "cheaters," that is, patients who were only improving their blood glucose prior to check up. HbA1c has also been used to diagnose anemia, reticulocytosis, polycythemia, and other diseases affecting the blood. Physicians now use HbA1c screening as a clinically effective means to evaluate risk of glycemic damage to tissues, particularly retinopathy, neuropathy, and nephropathy. Nonetheless, while meaningful to physicians and important to overall blood glucose control, the relationship between daily blood glucose testing and infrequent HbA1c assessment remains unclear to the average diabetic, yet increased awareness of the less labile picture of blood glucose afforded through HbA1c could benefit self management, especially between medical check ups.

Existing approaches to diabetes management still rely on physician decision-making. For instance, U.S. Patent No. 6,168,563, to Brown, discloses a healthcare maintenance system based on a hand-held device. Healthcare data, such as blood glucose, can be uploaded on to a program cartridge for healthcare professional analysis at a centralized location. Healthcare data can also be directly obtained from external monitoring devices, including blood glucose monitors. At the centralized location, blood glucose test results can be matched with quantitative information on medication, meals, or other factors, such as exercise. Changes in medication dosage or modification to the patient's monitoring schedule can be electronically sent back to the patient. However, decision making with respect to an insulin treatment regimen through interpretation of uploaded healthcare data remains an offline process, discretionary to and within the sole control and timing of the remote healthcare professional.

Similarly, U.S. Patent No. 6,024,699, to Surwit et al. ("Surwit"), discloses monitoring, diagnosing, prioritizing, and treating medical conditions of a plurality of remotely located patients. Each patient uses a patient monitoring system that includes medicine dosage algorithms, which use stored patient data to generate medicine dosage recommendations for the patient. A physician can modify the medicine dosage algorithms, medicine doses, and fixed or contingent self-monitoring schedules, including blood glucose monitoring through a central data processing system. In particular, diabetes patients can upload their data to the central data processing system, which will detect any trends or problems. If a problem is detected, a revised insulin dosing algorithm, insulin dosage, or self-monitoring schedule can be downloaded to their patient monitoring system. However, such modifications and revisions remain within the sole discretion and timing of a physician, who acts remotely via the central data processing system.

For the diabetic patient, guidance on how daily glucose control relates to HbA1c can help avoid longer term consequences from poor management. Therefore, there is a need for personal glucose management assistance, especially for Type 1 and Type 2 diabetics that is capable of adapting a regimen to on-going patient conditions in a localized and time-responsive fashion. Preferably, such assistance would be patient-operable and relate daily blood, interstitial, tissue, cellular, or other forms of glucose self-testing or automated testing results to an estimation of HbA1c.

## Summary

A system and method for modeling management of Type 1 or Type 2 diabetes mellitus on an individualized and continually fine-tunable basis is provided. An automated diabetes management tool is established by using the insulin, oral antidiabetic medication, and carbohydrate sensitivities of a diabetic as a reference starting point. Population-based insulin and oral antidiabetic medication activity curve data can be scaled to reflect the diabetic's personal sensitivities. A carbohydrate sensitivity can be determined through consumption of a standardized, timed test meal. A digestive response curve can be generated from the carbohydrate sensitivity by proportioning a time course curve based on postprandial blood glucose data, such as glycemic index. The personal insulin and oral antidiabetic medication activity curves and the personal digestive response curves form a personalized and automated diabetes management tool.

Additionally, a system and method for correlating daily glucose testing measurements to HbA1c estimates is provided. Glucose measurements are gathered over a backwards-looking time window, which is set to an adjustable period, typically 90 to 120 days. The glucose measurements can be obtained through blood, interstitial, tissue, cellular, or other testing. Interpolated glucose measurements are determined between pairs of the actual glucose measurements. An exponential decay function is projected over the time window to weight the glucose measurements according to the physiology of glucose binding to red cell proteins. Each glucose measurement, whether actual or interpolated, is multiplied by a decay constant corresponding to the point along the exponential decay function that the glucose measurement falls within the time period. The decayed glucose measurements are summed and multiplied by a scaling coefficient to yield an estimate of HbA1c. The estimate is accompanied by an ascription of accuracy, which reflects the degree to which the patient may rely on the estimate.

One embodiment provides a system and method for providing a personalized tool for estimating glycated hemoglobin. An electronically-stored history of empirically measured glucose levels for a patient is maintained over a set period of time in order of increasing age. A decay factor is applied to each of the measured glucose levels. The measured glucose levels are aggregated and scaled as decayed into an estimate of glycated hemoglobin for the time period. The glycated hemoglobin estimate is displayed to the patient.

A further embodiment provides a system and method for creating a personalized tool for estimating a time course of glucose effect for a diabetic patient. A patient history is stored and includes a multiplicity of empirically measured glucose levels for a diabetic patient ordered by increasing age. Regular intervals within the patient history are defined. Each of the measured glucose levels is assigned to the regular interval most closely corresponding to the age of the glucose level. An exponential decay function is projected over part of the patient history. Each of the measured glucose levels with the part of the patient history is adjusted by the exponential decay function. A summation of the adjusted measured glucose levels is taken and the summation into an estimate of glycated hemoglobin is scaled. The glycated hemoglobin estimate and the measured glucose levels included in the part of the patient history are displayed to the diabetic patient.

The personal predictive management tool provides diabetics with a new-found sense of personal freedom and safety by integrating the vagaries of daily glucose control into a holistic representation that can be continually re-evaluated and calibrated to keep pace with the unpredictable nature of daily life. Glucose testing is provided deeper meaning through real time estimation of HbA1c, which can also be applied in managing anemia, reticulocytosis, polycythemia, and other diseases affecting the blood, as well as diabetes. The approach described herein closely approximates what a normal pancreas does by interactively guiding the individual diabetic under consideration and, over time, learning how the patient can be understood and advised.

The present application extends to the following statements:

1. A system (330) for providing a personalized tool for estimating glycated hemoglobin, comprising:

a database (237) configured to maintain an electronically-stored history of empirically measured glucose levels (240) for a patient (21) over a set period of time in order of increasing age; and
a prediction module (332), comprising:

a decay module configured to apply a decay factor (334) to each of the measured glucose levels (240);
a scaling module configured to aggregate and scale the measured glucose levels (240) as decayed into an estimate of glycated hemoglobin (337) for the time period; and
a display module (234) configured to display the glycated hemoglobin estimate (337) to the patient (21).

2. A system (330) according to Statement 1, further comprising:

an estimation module (333), comprising:

an aggregation module configured to aggregate the measured glucose levels (240);
a scaling module configured to scale a summation of the measured glucose levels (240) as an estimate of accuracy of the glycated hemoglobin estimate (337),

wherein the display module (234) is further configured to display the estimate of accuracy to the patient (21).

3. A system (330) according to Statement 1, further comprising:

an interpolation module configured to interpolate estimated glucose levels (240) at each of the regular intervals along the time period between each pair of the measured glucose levels (240) in the history,
wherein the decay module is further configured to apply a decay factor (334) to each of the estimated glucose levels (240), and the scaling module is further configured to aggregate and scale the estimated glucose levels (240) as decayed with the measured glucose levels (240) as decayed into the estimate of glycated hemoglobin (337) for the time period.

4. A system (330) according to Statement 3, wherein each such estimated glucose level is established as one of a linear and an exponential interpolation of each such pair of measured glucose levels (240) maintained immediately adjacent to each other in the history.

5. A system (330) according to Statement 1, further comprising:

an exponential decay function defined beginning at a most recent point in the time period, wherein the decay factor (334) is chosen as a value of the exponential decay function corresponding to the regular interval for each of the measured glucose levels (240).

6. A system (330) according to Statement 1, wherein an exponential decay function is defined comprising fifty percent of the area of a curve projected over the first 30 days of the time period, wherein the time period comprises between 90 to 120 days.

7. A system (330) according to Statement 6, further comprising:

an exponential decay function *f(x)* expressed in accordance with:

$$f(x) = e^{-\lambda}$$

where *x* is one such regular interval, and λ comprises a decay constant between 40 percent and 60 percent.

8. A method (280) for providing a personalized tool for estimating glycated hemoglobin, comprising:

maintaining an electronically-stored history of empirically measured glucose levels (240) for a patient (21) over a set period of time in order of increasing age;

applying a decay factor (334) to each of the measured glucose levels (240);

aggregating and scaling the measured glucose levels (240) as decayed into an estimate of glycated hemoglobin (337) for the time period; and

displaying the glycated hemoglobin estimate (337) to the patient (21).

9. A method (280) according to Statement 8, further comprising:

aggregating the measured glucose levels (240);

scaling a summation of the measured glucose levels (240) as an estimate of accuracy of the glycated hemoglobin estimate (337); and

displaying the estimate of accuracy to the patient (21).

10. A method (280) according to Statement 8, further comprising:

interpolating estimated glucose levels (240) at each of the regular intervals along the time period between each pair of the measured glucose levels (240) in the history;

applying a decay factor (334) to each of the estimated glucose levels (240); and

aggregating and scaling the estimated glucose levels (240) as decayed with the measured glucose levels (240) as decayed into the estimate of glycated hemoglobin (337) for the time period.

11. A method (280) according to Statement 10, further comprising:

establishing each such estimated glucose level as one of a linear and an exponential interpolation of each such pair of measured glucose levels (240) maintained immediately adjacent to each other in the history.

12. A method (280) according to Statement 8, further comprising:

defining an exponential decay function beginning at a most recent point in the time period; and

choosing the decay factor (334) as a value of the exponential decay function corresponding to the regular interval for each of the measured glucose levels (240).

13. A method (280) according to Statement 8, further comprising:

defining an exponential decay function comprising fifty percent of the area of a curve projected over the first 30 days of the time period, wherein the time period comprises between 100 to 120 days.

14. A method (280) according to Statement 13, further comprising:

expressing the exponential decay function *f(x)* in accordance with:

$$f(x) = e^{-\lambda}$$

where *x* is one such regular interval, and λ comprises a decay constant between 40 percent and 60 percent.

Still other embodiments of the present invention will become readily apparent to those skilled in the art from the following detailed description, wherein are described embodiments by way of illustrating the best mode contemplated for carrying out the invention. As will be realized, the invention is capable of other and different embodiments and its several details are capable of modifications in various obvious respects, all without departing from the spirit and the scope of the present invention. Accordingly, the drawings and detailed description are to be regarded as illustrative in nature and not as restrictive.

## Brief Description of the Drawings

FIGURE 1 is a functional block diagram showing, by way of example, a prior art diabetes management cycle for a Type 1 diabetic.

FIGURE 2 is a functional block diagram showing, by way of example, an automated diabetes management cycle for a Type 1 diabetic, in accordance with one embodiment.

FIGURES 3A-C are functional block diagrams showing, by way of example, prior art diabetes management cycles for a Type 2 diabetic.

FIGURES 4A-C are functional block diagrams showing, by way of example, automated diabetes management cycles for a Type 2 diabetic, in accordance with one embodiment.

FIGURE 5 is a process flow diagram showing personalized Type 1 and Type 2 diabetes mellitus modeling.

FIGURE 6 is a diagram showing, by way of example, a screen shot of a graphical user interface for establishing a personalized diabetes management tool for Type 1 and Type 2 diabetes mellitus.

FIGURE 7 is a diagram showing, by way of example, a screen shot of a graphical user interface for specifying insulin preparation type for use in the graphical user interface of FIGURE 6.

FIGURE 8 is a diagram showing, by way of example, a screen shot of a graphical user interface for specifying other medications for use in the graphical user interface of FIGURE 6.

FIGURE 9 is a diagram showing, by way of example, a screen shot of a graphical user interface for selecting food combinations for use in the graphical user interface of FIGURE 6.

FIGURE 10 is a process flow diagram showing a method for establishing a personalized diabetes management tool for Type 1 and Type 2 diabetes mellitus, in accordance with one embodiment.

FIGURE 11 is a process flow diagram showing a routine for establishing an insulin activity curve for use with the method of FIGURE 10.

FIGURE 12 is a process flow diagram showing a routine for calibrating an insulin activity curve for use with the method of FIGURE 10.

FIGURE 13 is a process flow diagram showing a routine for establishing an oral antidiabetic medication activity curve for use with the method of FIGURE 10.

FIGURE 14 is a process flow diagram showing a routine for calibrating an oral antidiabetic medication activity curve for use with the method of FIGURE 10.

FIGURE 15 is a graph showing, by way of example, an insulin activity curve for lispro, an insulin analog.

FIGURE 16 is a diagram showing, by way of example, a screen shot of a personal insulin activity curve for display in the graphical user interface of FIGURE 6.

FIGURE 17 is a process flow diagram showing a routine for establishing a digestive response curve for use with the method of FIGURE 10.

FIGURE 18 is a graph showing, by way of example, a personal digestive response curve for a standardized meal.

FIGURE 19 is a process flow diagram showing, by way of example, characteristics affecting diabetes management.

FIGURE 20 is a process flow diagram showing, by way of example, factors bearing on personal predictive diabetes management.

FIGURE 21 is a block diagram showing for a system for generating a personalized diabetes management tool for Type 1 diabetes mellitus, in accordance with one embodiment.

FIGURE 22 is a graph showing, by way of example, mean blood glucose profile compared to HbA1c level over a set time period.

FIGURE 23 is a diagram showing, by way of example, a screen shot of a graphical user interface for providing an estimate of HbA1c for use in the graphical user interface of FIGURE 6.

FIGURE 24 is a process flow diagram a method for providing a personalized tool for estimating glycated hemoglobin in accordance with a further embodiment.

FIGURE 25 is a flow diagram showing a routine for estimating HbA1c for use with the method of FIGURE 24.

FIGURE 26 is a graph showing, by way of example, an exponential decay function projected over a set time period.

FIGURE 27 is a flow diagram showing a routine for estimating accuracy for use with the method of FIGURE 24.

FIGURE 28 is a block diagram showing for a system for providing a personalized tool for estimating glycated hemoglobin, in accordance with a further embodiment.

**Detailed Description**

Diabetes Management Cycles

Both Type 1 and Type 2 diabetes are diseases that require continuous and consistent glucose management. Poorly controlled diabetes affects both quality of life and longevity, which can be dramatically curtailed due to avoidable chronic complications. Conversely, acute conditions will occur with even well-managed patients, although proper management helps to significantly lower the likelihood.

Type 1 Diabetes

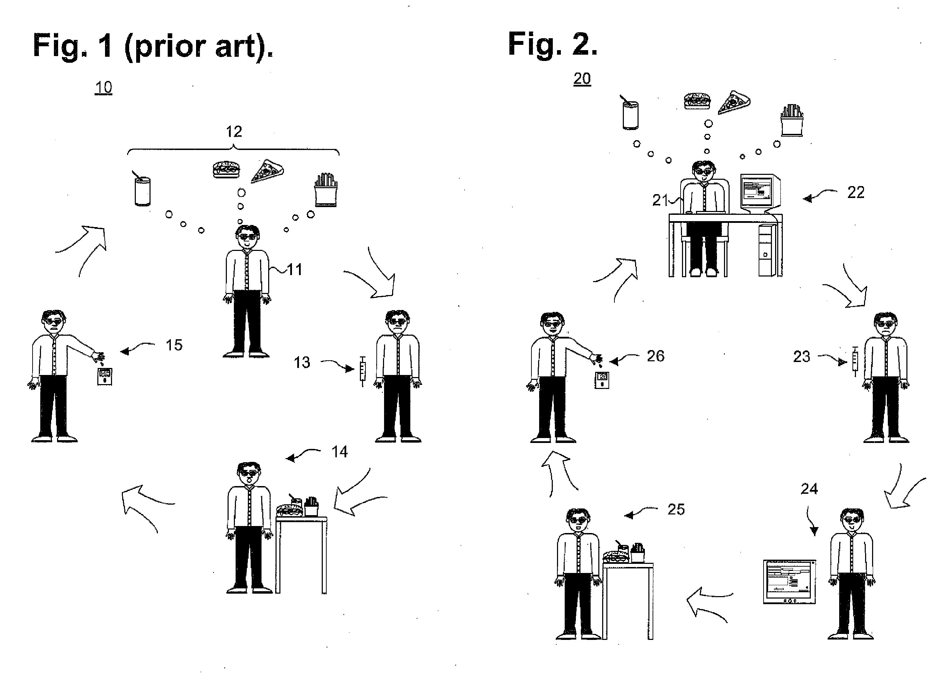

The principal cause of Type 1 diabetes is a T-cell mediated autoimmune attack on the beta cells of the islets of Langerhans of the pancreas. No known preventative measures exist. Type 1 diabetes management is a continual process that is repeated on a daily basis. FIGURE 1 is a functional block diagram showing, by way of example, a prior art diabetes management cycle 10 for a Type 1 diabetic. Fundamentally, Type 1 diabetes management centers on the timing and content of meals and the timing and dosing of insulin, although other factors, such as physical activity and exercise, overall physical well-being, other illnesses, and stress, can influence the course of management.

Currently, Type 1 diabetes can only be treated through insulin therapy, which is normally combined with adjustments to patient lifestyle, including diet and exercise. As a result, a Type 1 diabetic patient 11 learns to plan and time his daily meals (step 12) to estimate an expected rise in blood glucose and to determine appropriate doses of insulin to counteract the expected rise.

Generally, a Type 1 diabetic administers insulin prior to actually consuming any food (step 13). A post-meal increase in blood glucose is normal, but the insulin in intended to bring blood glucose back down to a reasonable range within two to four hours. A Type 1 diabetic determines the insulin units needed to counteract an expected post-meal rise in blood glucose and times his insulin to counteract the affect of the meal (step 14). Ideally, a Type 1 diabetic's average blood glucose should be in the range of 80-120 mg/dL, although a range of 140-150 mg/dL is often used to prevent potentially life-threatening hypoglycemic events. In effect, long-term management of blood glucose levels is short-changed to prevent the more pressing short-term consequences of hypoglycemia.

Physicians encourage each Type 1 diabetic to regularly self-test his blood glucose (step 15) to enable better compensation for patient-specific sensitivities to both food and insulin. A patient 11 places a drop of blood on a test strip coated with a glucose oxidase or hexokinase enzyme, which is read by a glucose monitor. Blood glucose is normally tested daily, although stricter control regimens may require more frequent testing.

Patient logs document the interaction of food, insulin, and patient sensitivities. Physician review normally only occurs during clinic visits, or when otherwise necessary. Consequently, detailed context is lost, unless the patient comprehensively records exacting descriptions of all food components consumed and their manner of preparation, precise times between insulin dosing and completion of a meal, physiological factors, such as mood or wellness, and similar data. The physician must be willing to study a patient log in corresponding detail. However, neither detailed patient documentation nor close physician review are practical in terms of time, effort, and cost for every Type 1 diabetic patient.

The accuracy and timeliness of a Type 1 diabetes management regimen can be improved by automating the predictive aspects of glycemic control. FIGURE 2 is a functional block diagram showing, by way of example, an automated diabetes management cycle 20 for a Type 1 diabetic, in accordance with one embodiment. Automation is introduced to move the management cycle significantly closer to a closed loop arrangement. Control is streamlined and steps that remain to be performed by a patient manually are minimized, or even eliminated, which make such steps less apt to be forgotten or missed.

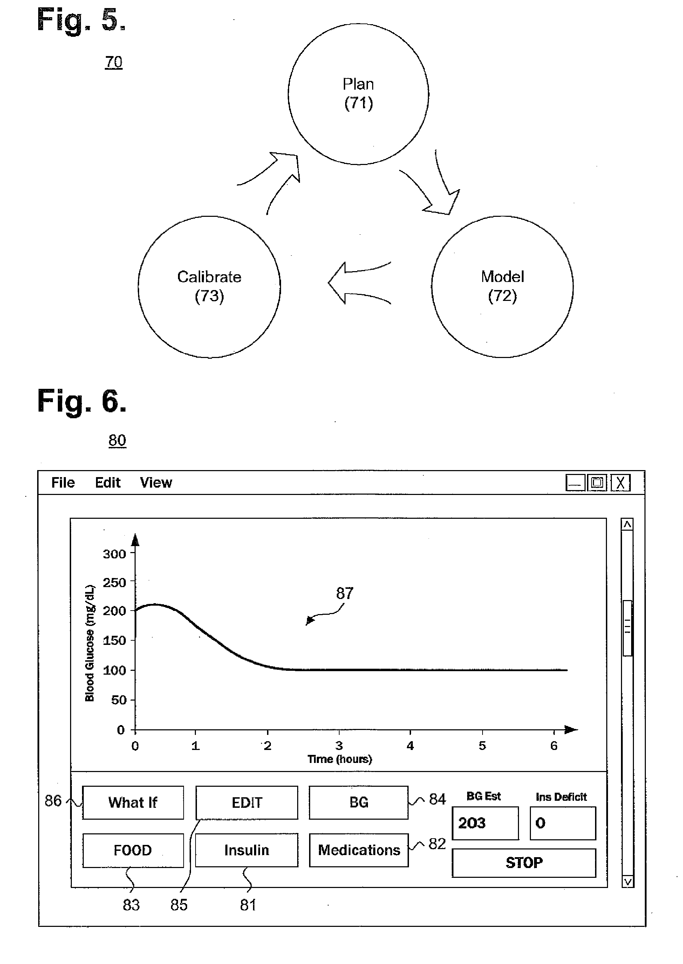

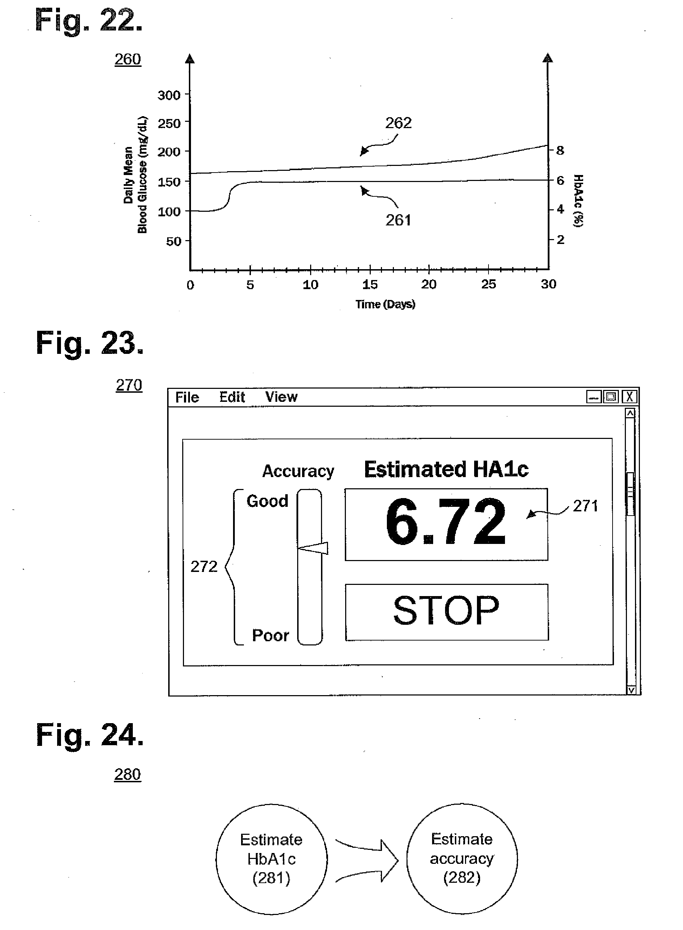

An automated diabetes management tool applies heuristics to model and calibrate a personalized diabetes control regimen for a Type I diabetic patient 21 (step 22), as further described below beginning with reference to FIGURE 5. Through the management tool, the patient 21 can plan and time insulin dosing and meals (steps 23 and 25, respectively) more accurately than possible through conventional means, including exchange lists and carbohydrate counting. Dynamically tracked blood glucose predictions (step 24) can also be provided and self-testing results (step 26) can be provided directly into the management tool for time-responsive integration and evaluation. In a further embodiment, HbA1c estimates are generated from the self-testing results, as further described below beginning with FIGURE 22. In a still further embodiment, emergent glucose self-testing, such as interstitial, tissue, or cellular glucose testing, supplements manual self-testing or, where sufficiently reliable, replaces manual self-testing to achieve a fully closed loop system when combined with insulin pump therapy. Other management tool aspects are possible.

Type 2 Diabetes

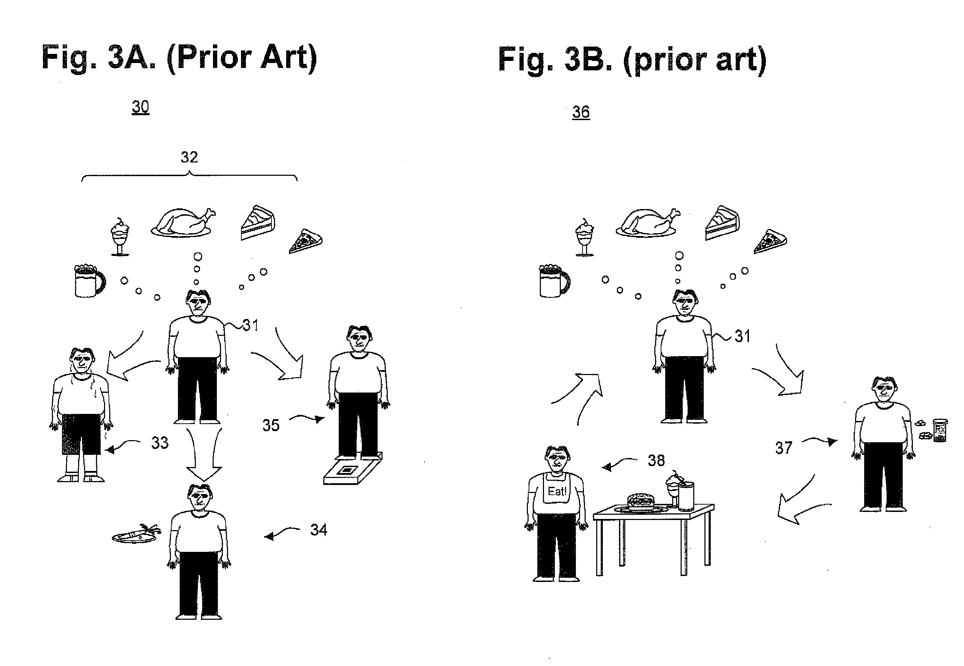

Type 2 diabetes is due to defective insulin secretion, insulin resistance, or reduced insulin sensitivity. As with Type 1 diabetes, no known preventative measures exist, but strong correlations to obesity and genetic predisposition have been observed. Like Type 1 diabetes, Type 2 diabetes management is a continual process with the nature and magnitude of management interventions progressing over time. FIGURES 3A-C are functional block diagrams showing, by way of example, prior art diabetes management cycles for a Type 2 diabetic. The earliest stage of Type 2 diabetes can be controlled through lifestyle changes alone, after which antidiabetic medications and ultimately insulin therapy are eventually added.

Early stage Type 2 diabetes management focuses on changes in lifestyle with emphasis on basic glycemic control. Referring first to FIGURE 3A, a typical patient 31 is often obese, although obesity is but one indicator of Type 2 diabetes, which further includes genetic predisposition and mutation of amylin genes. Diet 32 often plays a significant role. As a result, the patient 31 is urged to exercise, for instance, by taking a brisk 45-minute walk several times a week, and to increase his physical activity (step 33) through a combination of aerobic and resistance training. In addition, the patient 31 is educated on following a healthy diet (step 34) to decrease his weight (step 35), because the level of insulin resistance proportionately grows with the increase in body fat, particularly metabolically active visceral fat.

Effective early stage Type 2 diabetes control can temporarily restore normal insulin sensitivity, although the predisposition for insulin resistance remains. Referring next to FIGURE 3B, oral antidiabetic medications are generally prescribed (step 37) as insulin production becomes impaired, yet partial pancreatic function remains. Most commonly, beguanide metformin and sulfonylureas are prescribed to respectively help regulate inappropriate hepatic glucose release and stimulate insulin production. Thiazolidinediones may also be prescribed, which decrease insulin resistance. Recommendations regarding lifestyle changes (step 38) in exercise, diet, and weight loss continue.

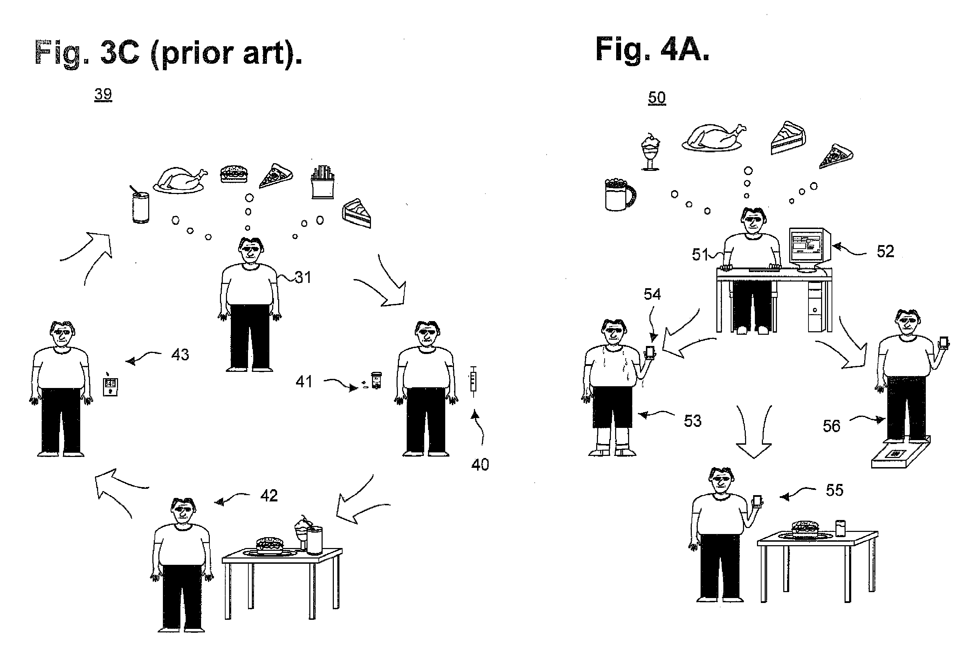

In the last stage, pancreatic function ceases altogether, which necessitates commencement of insulin therapy. Referring to FIGURE 3C, insulin is administered subcutaneously (step 40). Insulin therapy is performed in a manner similar to a Type 1 diabetic, which includes both meal and insulin dosage planning, as described above with reference to FIGURE 1. However, oral antidiabetic medications (step 41) may also be taken, along with continued adherence to lifestyle changes (step 42). Additionally, the patient 31 is now encouraged to self-test his blood glucose (step 43).

Many aspects of Type 2 diabetes management can also be automated. FIGURES 4A-C are functional block diagrams showing, by way of example, automated diabetes management cycles for a Type 2 diabetic, in accordance with one embodiment. In a manner similar to Type 1, automation is introduced to increase the accuracy and timeliness of blood glucose control and data logging, and to minimize or eliminate steps performed by a patient manually.

Type 2 diabetes is a progressively debilitating disorder and quality of life can best be preserved by seeding diabetes awareness from the earliest stages of the disease. Referring first to FIGURE 4A, a Type 2 diabetic patient 51 faces making changes to his lifestyle through physical activity (step 53), diet (step 55), and weight (step 56). Hopefully, the changes are permanent, but a diabetic 51 may lose sight of their importance, either through indifference or by temporary restoration of insulin sensitivity. Consequently, during early stage Type 2 diabetes, an automated diabetes management tool can be used to assist the patient 51 in planning his diet and physical activities (step 52), and in tracking his progress (step 54) for subsequent review and analysis, as further described below beginning with reference to FIGURE 5.

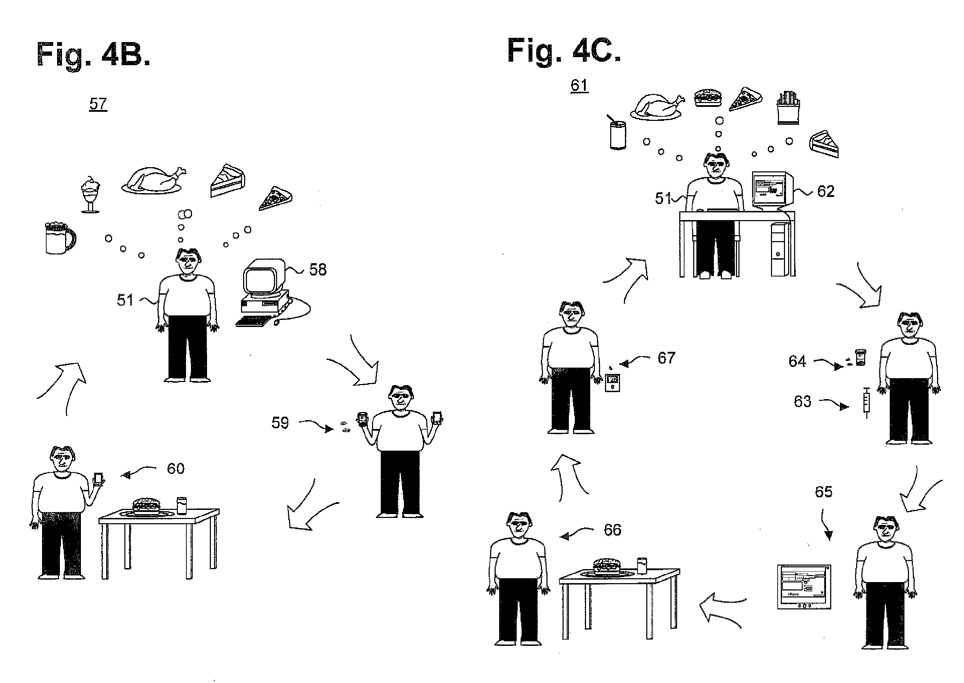

As insulin resistance increases and pancreatic function decreases, oral antidiabetic medications become increasingly important. Referring next to FIGURE 4B, the types and timing of medications required will depend upon the patient's physiology and physical tolerance. Moreover, medication may be necessary at different times of the day and in different combinations. In addition to planning (step 58) and tracking (step 60) functions, the management tool can also provide dosing instructions and reminders (step 59) to guide the patient 51 in therapy compliance.

End-stage Type 2 diabetes will require insulin therapy. Referring finally to FIGURE 4C, use of insulin requires conscious planning (step 62) and conscientious dosing (step 63), both in appropriate amount and at the correct time with respect to anticipated meals to effectively lower the blood sugar and to prevent the adverse consequences of hypoglycemia. The management tool applies planning (step 62) and tracking (step 66) functions similar in form to the methodology of Type 1 diabetes, but further includes administration of oral antidiabetic medications (step 64). In addition, the management tool can provide dynamic blood glucose prediction (step 65) and blood glucose self-testing integration (step 67). In a further embodiment, HbA1c estimates are generated from the self-testing results, as further described below beginning with FIGURE 22. In a still further embodiment, interstitial, tissue, or cellular glucose testing and similar diagnostics supplement or replace manual self-testing, which provide a fully closed loop system when combined with a wireless insulin pump. Other management tool aspects are possible.

Automated Management of Type 1 and Type 2 Diabetes

The diabetic patient is himself the best resource available to manage diabetes. Meals, insulin dosing, and changes in personal well being, as well as departures from such plans, are best known to the patient, who alone is ultimately responsible for adherence to a management regimen. FIGURE 5 is a process flow diagram showing personalized Type 1 and Type 2 diabetes mellitus modeling 70. The method is performed as a series of process steps or operations executed by one or more patient-operable devices, including personal computers, personal digital assistants, programmable mobile telephones, intelligent digital media players, or other programmable devices, that are working individually or collaboratively to apply a personal predictive management tool.

Modeling involves projecting the glycemic effect of planned meals in light of insulin dosing, if applicable, and oral antidiabetic medications (primarily Type 2). Meal planning is particularly important to Type 1 and end-stage Type 2 diabetics, where the content and timing of meals greatly impacts blood glucose and must be closely controlled by dosed insulin to compensate for the lack of naturally-produced insulin. Dietary management is less crucial to non-end-stage Type 2 diabetics, who still retain limited natural insulin production. Nevertheless, proper diet can aid with weight control and hepatic glucose release. For all Type 1 and Type 2 diabetics, the management tool performs dietary planning (step 71), which primarily involves determining the glycemic effect of food based on a standardized meal. In a further embodiment, planning also includes projecting the effect of exercise or physical activities that are likely to require appreciable caloric expenditure. Other planning aspects are possible.

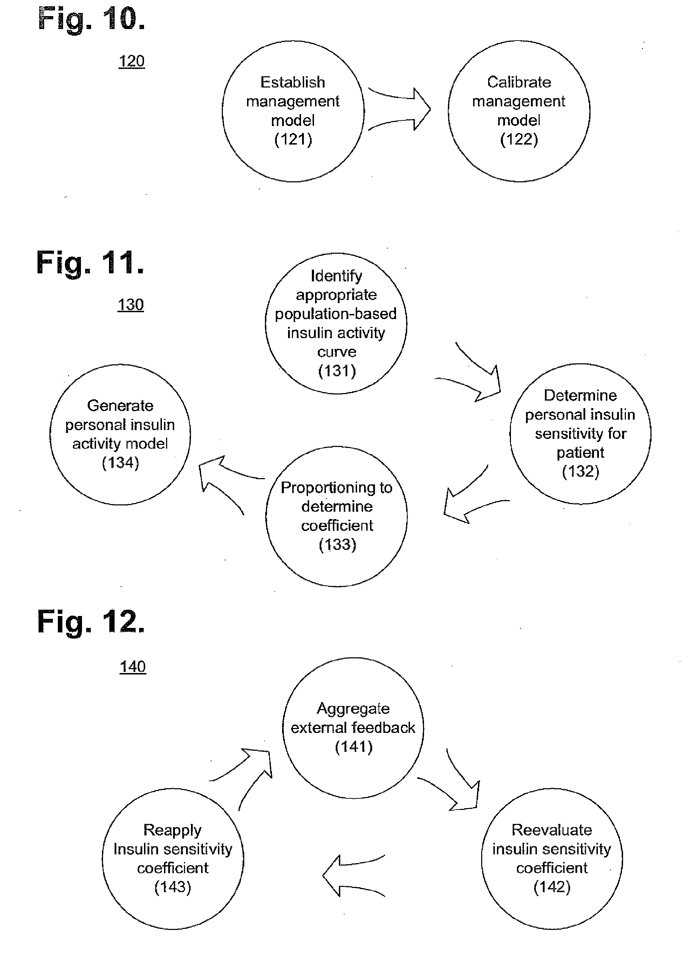

Once each planned meal is known, the management tool can model the time courses and amplitudes of change for the meal, dosed insulin, and oral antidiabetic medication (primarily Type 2) (step 72). Additionally, the management tool can be calibrated as necessary to adjust to self-testing and data recorded by the patient (step 73), as further described below beginning with reference to FIGURE 10. Other modeling and calibration aspects are possible.

In a further embodiment, the patient can combine different food types and quantities and perform "What If" scenarios as an aid to blood glucose management. The physiological effects on blood glucose of specific food and beverage, both individually and in combination, are modeled, taking into account differences in digestive motility and other factors, such as described in commonly-assigned U.S. Patent application, entitled "System And Method For Actively Managing Type 1 Diabetes Mellitus On A Personalized Basis," Serial No. 12/030,087, filed February 12, 2008, pending; U.S. Patent application, entitled "System and Method for Managing Type 1 Diabetes Mellitus Through a Personal Predictive Management Tool," Serial No. 12/030,120, filed February 12, 2008, pending, U.S. Patent application, entitled "System And Method For Actively Managing Type 2 Diabetes Mellitus On A Personalized Basis," Serial No. 12/030,097, filed February 12, 2008, pending; U.S. Patent application, entitled "System and Method for Managing Type 2 Diabetes Mellitus Through a Personal Predictive Management Tool," Serial No. 12/030,130, filed February 12, 2008, pending, the disclosures of which are incorporated by reference. The modeling is based on an individual patient's personal dietary tastes and preferences and the blood glucose rises that ensue following consumption, as well as capturing the synergies and interactions of various food preparations and combinations.

Graphical User Interface

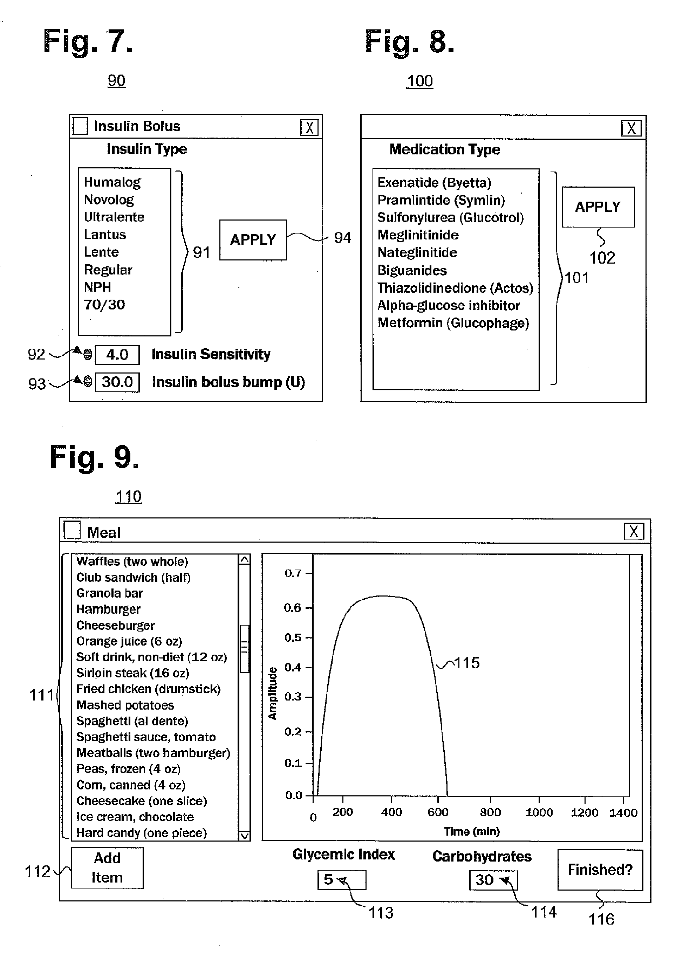

Personalized Type 1 and Type 2 diabetes mellitus modeling can be provided through a patient-operable interface through which planning and calibration can be performed. FIGURE 6 is a diagram showing, by way of example, a screen shot of a graphical user interface 80 for establishing a personalized diabetes management tool for Type 1 and Type 2 diabetes mellitus, in accordance with one embodiment. The user interface 80 provides logical controls that accept patient inputs and display elements that present information to the patient. The user interface 80 can be used for both Type 1 and Type 2 diabetes management, although the applicability of particular logical controls, screens, and menus will depend upon the type and stage of the patient's diabetes. The logical controls include buttons, or other forms of option selection, to access further screens and menus to specify an insulin bolus 81 ("Insulin"), as further described below with reference to FIGURE 7; specify other oral antidiabetic medications 82 ("Medications") (primarily Type 2), as further described below with reference to FIGURE 8; plan meals 83 ("FOOD"), as further described below with reference to FIGURE 9; enter a measured blood glucose reading 84 ("BG"); edit information 85 ("EDIT"); and speculate on what would happen if some combination of food, insulin, or antidiabetic or oral medication (primarily Type 2) were taken 86 ("What If"). Further logical control and display elements are possible.

To assist the patient with planning, a graphical display provides a blood glucose forecast curve 87, which predicts combined insulin dosing, oral antidiabetic medication administration (primarily Type 2), and postprandial blood glucose. The $x$-axis represents time in hours and the $y$-axis represents the blood glucose level measured in mg/dL. Modeling estimates the timing and amplitude of change in the patient's blood glucose in response to the introduction of a substance, whether food, physiological state, or drug, that triggers a physiological effect in blood glucose. Generally, actions, such as insulin dosing, medication administration, exercise, and food consumption cause a measurable physiological effect, although other substances and events can influence blood glucose. The time courses and amplitudes of change are adjusted, as appropriate, to compensate for patient-specific factors, such as level of sensitivity or resistance to insulin, insulin secretion impairment, carbohydrate sensitivity, and physiological reaction to medications. Other patient-specific factors, like exercise or supervening illness, may also alter the time courses and amplitudes of blood glucose.

In one embodiment, the user interface 80 and related components are implemented using a data flow programming language provided through the LabVIEW development platform and environment, licensed by National Instruments Corporation, Austin, TX although other types and forms of programming languages, including functional languages, could be employed. The specific option menus will now be discussed.

Insulin Selection

When insulin therapy is applicable, such as for a Type 1 or end-stage Type 2 diabetic, a patient needs to identify both the type and amount of insulin preparation used and his sensitivity to allow the management tool to generate an insulin response curve. Insulin preparation types are identified by source, formulation, concentration, and brand name, and are generally grouped based on duration of action. FIGURE 7 is a diagram showing, by way of example, a screen shot of a graphical user interface 90 for specifying insulin preparation type for use in the graphical user interface 80 of FIGURE 6. Different types of insulin preparation 91 can be selected and, for ease of use and convenience, are identified by brand name or formulation. Insulin preparations include short-acting insulins, such as lispro or Regular, that are used to cover a meal and are frequently administered by insulin pump due to the short time of onset. Intermediate-acting insulins, such as NPH (Neutral Protamine Hagedorn), have 12-18 hour durations, which peak at six to eight hours. Finally, long-acting insulins, such as Ultralente, have 32-36 hour durations to provide a steady flow of insulin. Long-acting insulins are generally supplemented with short-acting insulins taken just before meals. Other types of insulin preparations include insulin glargine, insulin detemir, and insulin preparation mixes, such as "70/30," which is a premixed insulin preparation containing 70% NPH insulin and 30% Regular insulin. In addition, insulin sensitivity 92 and an insulin bolus "bump" 93, that is, a single dosing, such as for meal coverage, is specified, before being factored into the tool upon pressing of the "APPLY" button 94. Further logical control and display elements are possible.

Other Medication Selection (Primarily Type 2)

Type 2 diabetics generally start with oral antidiabetic medications and only later progress to insulin therapy as insulin production ceases. However, both Type 1 and Type 2 diabetics may receive other medications in addition to insulin and antidiabetic oral medications. Each non-diabetic medication should also be identified to allow the management tool to project any effect on glycemic activity. FIGURE 8 is a diagram showing, by way of example, a screen shot of a graphical user interface 100 for specifying other medications for use in the graphical user interface of FIGURE 6. Different medications 101 can be selected and, for ease of use and convenience, can be identified by generic name, brand name, or formulation. As appropriate, the therapeutic effects, particularly as relating to blood glucose level, and drug interactions of each medication can be factored into the tool upon pressing of the "APPLY" button 102. For example, pramlintide acetate, offered under the brand name Symlin, is prescribed to both Type 1 and Type 2 diabetics to help lower postprandial blood glucose during the three hours following a meal. Consequently, the blood glucose rise is adjusted to reflect the effects of the pramlintide acetate in light of a planned meal and dosed insulin, if applicable. Further logical control and display elements are possible.

Food Selection

Unlike insulin preparation or other medications, the possible selections and combinations of food and beverage are countless and applicable, whether Type 1 or Type 2 diabetic, regardless of disease state. Moreover, how a particular food combination synergistically acts is equally variable. FIGURE 9 is a diagram showing, by way of example, a screen shot of a graphical user interface 110 for selecting food combinations for use in the graphical user interface 80 of FIGURE 6. Dietary management, and thence, the management tool, focuses on carbohydrates, which have the greatest affect on blood glucose. Simple sugars increase blood glucose rapidly, while complex carbohydrates, such as whole grain bread, increase blood glucose more slowly due to the time necessary to break down constituent components. Fats, whether triglyceride or cholesterol, neither raise nor lower blood glucose, but can have an indirect affect by delaying glucose uptake. Proteins are broken down into amino acids, which are then converted into glucose that will raise blood glucose levels slowly. Proteins will not generally cause a rapid blood glucose rise. Nevertheless, both fats and proteins are incorporated into the model by virtue of their empiric effect on blood glucose levels. Additionally, various combinations or preparations of medications or food can have synergistic effects that can alter blood glucose rise and timing.

In the management tool, the food choices 111 are open-ended, and one or more food item can be added to a meal by pressing the "ADD ITEM" button 112. Glycemic effect data, such as the glycemic index 113 and carbohydrates type and content 114 for a particular food item, are also displayed. A cumulative digestive response curve 115 is generated and is mapped to run contemporaneous to the insulin activity curve, so the affect of an insulin dose can be weighed against food ingestion. The cumulative digestive response curve 115 is based on the selections made by proportionately applying the patient's carbohydrate sensitivity. For instance, the selection of a 12-ounce non-diet soft drink and a 16-ounce sirloin steak would result in a cumulative digestive response curve with an initial near term peak, which reflects the short time course and high glucose content of the soft drink, and a long term peak, which reflects the protein-delayed and significantly less-dramatic rise in blood glucose attributable to the sirloin steak. The completion of meal planning is indicated by pressing the "Finished" button 116. Further logical control and display elements are possible.

Method

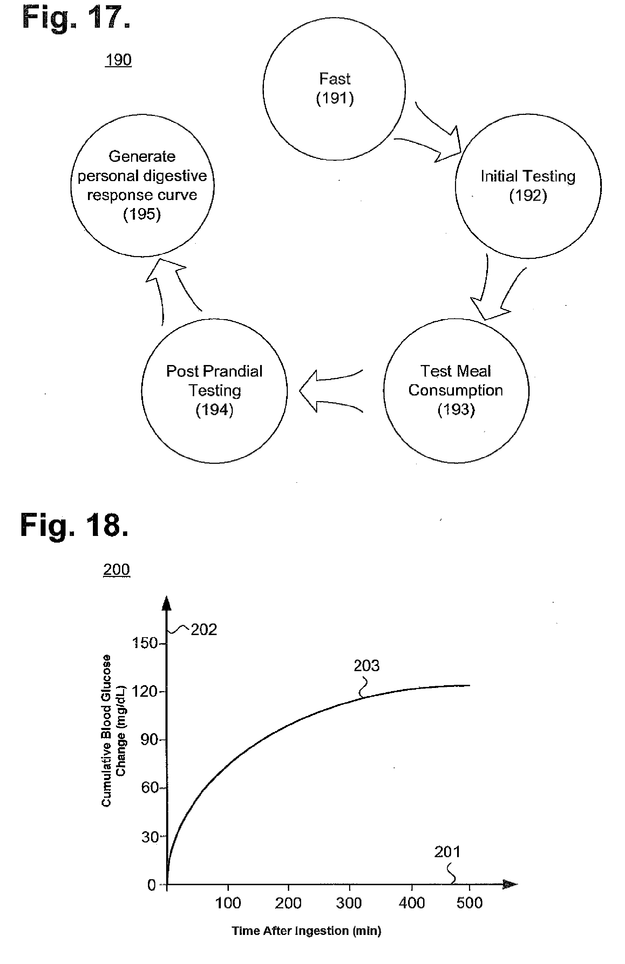

Conventional Type 1 and Type 2 diabetes management is predicated on application of population-based norms, which can serve as a starting point for personalized care. Individualized diabetes management adapts these norms to a model to meet specific patient needs and sensitivities and the model can be continually updated and fine tuned to address dynamic conditions. FIGURE 10 is a process flow diagram showing a method 120 for establishing a personalized diabetes management tool for Type 1 and Type 2 diabetes mellitus, in accordance with one embodiment. The method first establishes a personal predictive management tool (operation 121). One aspect of the model is determined for insulin activity, as further described below with reference to FIGURE 11, and a second aspect is determined for digestive speed and amplitude, as further described below with reference to FIGURE 17. Other aspects of the management tool are possible.

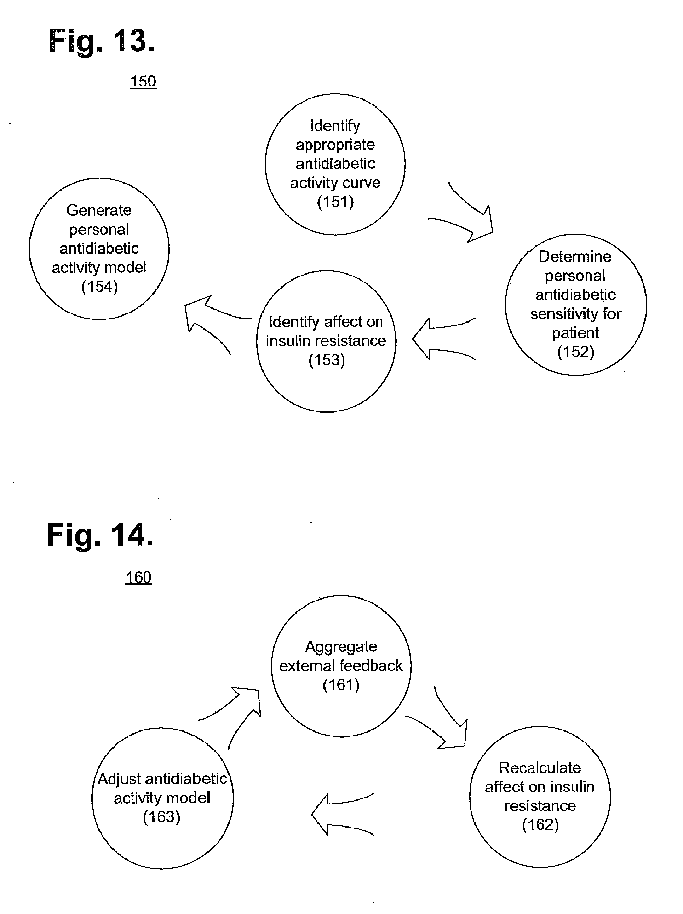

Once established, the management tool can be refined and calibrated on an on-going basis (operation 122) by integrating self-testing and other patient data sources in a localized and time-responsive fashion, as further described below with reference to FIGURE 13 for insulin activity. Through calibration, the management tool continually changes as more data is obtained and keeps pace with the patient over time.

Insulin Activity Modeling

Insulin is dosed in Type 1 and end-stage Type 2 diabetics to counteract the postprandial rise in blood glucose. Insulin activity is initially modeled with an insulin activity curve for a patient population as published for a specific insulin preparation. The insulin activity curve is adapted to each specific patient by factoring individual sensitivities into the personal predictive management tool.

In a further embodiment, the management tool requires the inclusion of dosed insulin for Type 1 and end-stage Type 2 diabetics. Requiring the modeling of an activity time curve for dosed insulin is particularly important when antidiabetic or oral medications are also modeled, as the latter can skew blood glucose and cause an incomplete impression of glycemic effect if dosed insulin is omitted from the model.

Establishing an Insulin Activity Curve

The clinical pharmacologies of various types of insulin are widely available from their manufacturer. The three major manufacturers of insulin are Eli Lilly, Novo Nordisk, and Sanofi Aventis, although other manufacturers exist. Each pharmacology typically includes a time course of action based on population-based clinical studies. Time courses are provided as general guidelines, which can vary considerably in different individuals or even within the same individual depending upon activity and general health. Time courses can serve as the basis of a management tool. FIGURE 11 is a process flow diagram showing a routine 130 for establishing an insulin activity curve for use with the method 120 of FIGURE 10. A model is generated for a specific type of insulin preparation. Similar models for other insulin preparation types can be postulated by extension, or established individually on a case-by-case basis.

Initially, a population-based insulin activity curve that is appropriate to a particular patient is identified (operation 131). A published time course of action for the insulin preparation type can be used, which provides an established baseline for insulin activity that can be adapted to the patient. Other sources of insulin activity curves can be used, so long as the curve accurately reflects time of onset, peak time, duration, or other essential insulin activity characteristics.

The personal level of sensitivity to the insulin preparation must also be determined for the patient (operation 132). Personal insulin sensitivity can be determined empirically, such as taking an empirically observed decrease in blood glucose for a fixed dose of the insulin preparation as the insulin sensitivity. In a further embodiment, personal insulin sensitivity can be determined by adapting interstitial, tissue, or cellular glucose levels for a fixed dose of the insulin preparation to the blood glucose level. Other determinations of personal insulin sensitivity are possible, including clinically-derived values.

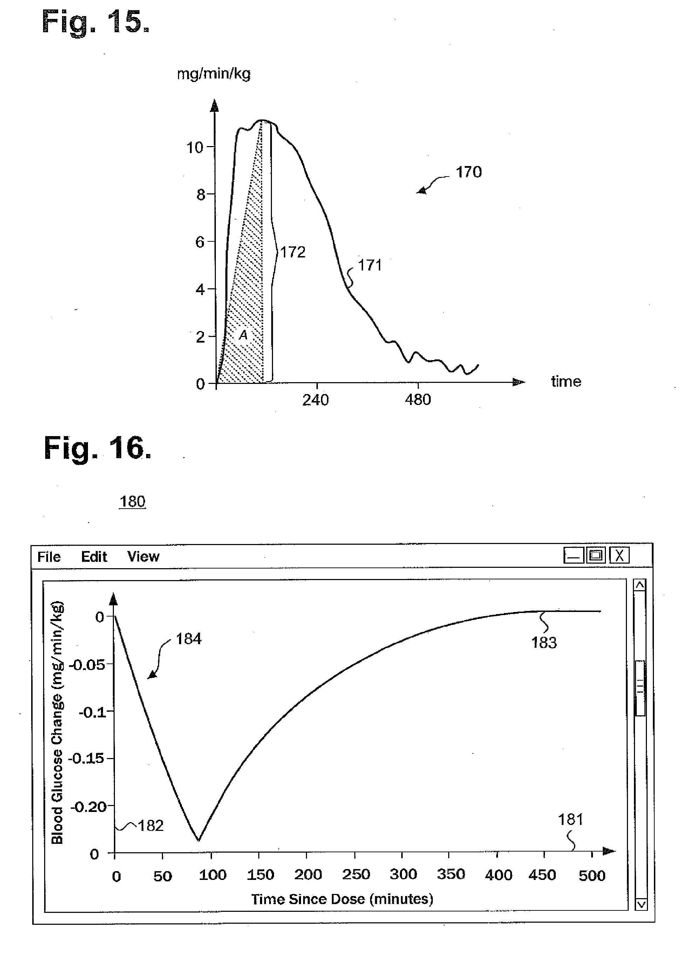

Based on the personal insulin sensitivity, an insulin sensitivity coefficient or coefficients can be found by proportioning the personal insulin sensitivity to the population-based insulin activity curve (operation 133). The coefficient can be determined through area estimation, as further described below with reference to FIGURE 15. Finally, once determined, the coefficient can be applied to the population-based insulin activity curve to generate a personal insulin activity model (operation 134). An application of an insulin sensitivity coefficient is further described below with reference to FIGURE 16.

Calibrating an Insulin Activity Curve

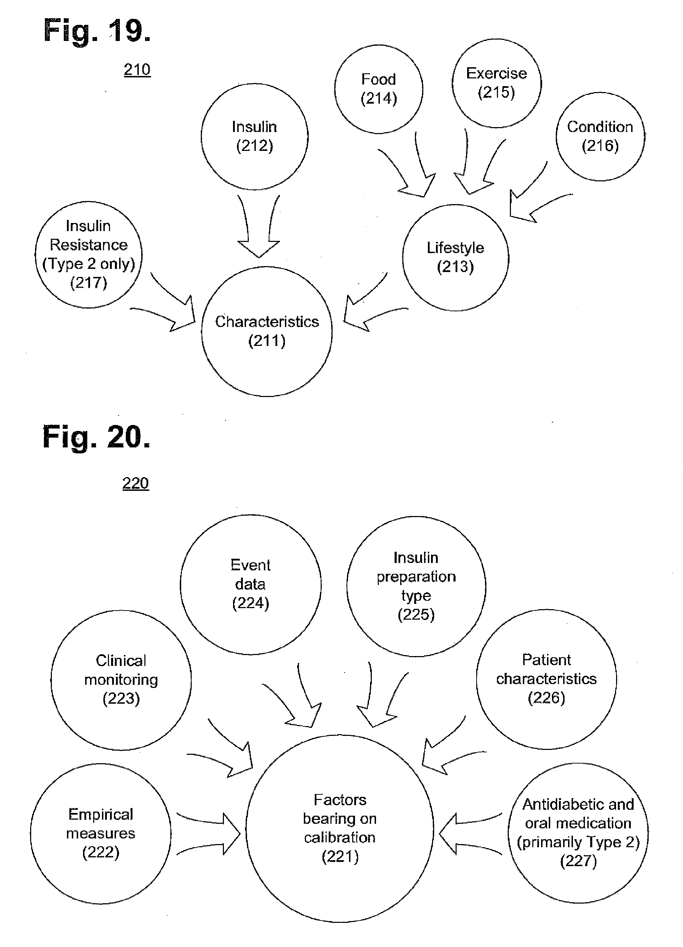

Diabetes management needs can change over time, as dietary habits, personal well being, and other factors occur in a diabetic's life. Thus, the management tool accommodates evolving conditions to remain current and to continue to provide effective guidance. FIGURE 12 is a process flow diagram showing a routine 140 for calibrating an insulin activity curve for use with the method 120 of FIGURE 10. Calibration is a dynamic process that builds on conventional self-testing and diabetes control information ordinarily only recorded as static data for potential physician consideration.
Calibration can be performed regularly, or only as needed. Various concerns can change how a management tool is characterized for a Type 1 diabetic, including factors relating to insulin, oral antidiabetic medication, and lifestyle, as further described below with reference to FIGURE 19. During each calibration, external feedback regarding the dosed insulin is aggregated into the management tool (operation 141) and applied to re-evaluate the insulin sensitivity coefficient (operation 142). The insulin sensitivity coefficient is affected only where the feedback reflects a non-nominal departure from an earlier provided sensitivity. A non-nominal departure occurs, for instance, when an observed decrease in blood glucose differs from a predicted blood glucose decrease by one or more standard deviations, although other thresholds or metrics of significance are possible. In addition, the feedback can be aggregated over several sample sets, or types of feedback, as further described below with reference to FIGURE 20. A revised personal insulin activity model can then be generated (operation 143) as a reflection of current circumstances.

Oral Antidiabetic Medication Activity Modeling (Primarily Type 2)

Oral antidiabetic medications are prescribed only to Type 2 diabetics during the middle and final stages of the disease and are selectively used with Type 1 diabetics. The type of effect on blood glucose depends upon the drug's pharmacology and the patient's sensitivity to the drug. For example, a meglitinide stimulates the release of natural insulin, which has the affect of directly lowering blood glucose. In contrast, thiazolidinediones increase insulin receptivity by stimulating glucose update, which indirectly lowers blood glucose, but is also dependent upon patient's insulin sensitivity. As a result, an activity curve can be generated based on population-based studies, but will ordinarily require adjustment in the management tool for each patient.

Establishing an Oral Antidiabetic Medication Activity Curve

Like insulin, the clinical pharmacologies of the different varieties of oral antidiabetic medications are widely available from their manufacturer and typically include a time course of action based on population-based clinical studies. In general, the population-based time courses can serve as the initial basis of a management tool. FIGURE 13 is a process flow diagram showing a routine 150 for establishing an oral antidiabetic medication activity curve for use with the method 120 of FIGURE 10. A model is generated for each antidiabetic or oral medication identified by the patient. Similar models for other medications can be postulated by extension, or established individually on a case-by-case basis.
Initially, a population-based activity curve that is appropriate to a particular patient is identified (operation 151). A published time course of action can be used. In a further embodiment, an empirical time course of action is determined by monitoring the patient's blood glucose following dosing of the medication. Other sources of activity curves can be used, which accurately reflect time of onset, peak time, duration, or other essential activity characteristics.
The patient's level of sensitivity to the medication is also determined (operation 152), which can be found empirically. In a further embodiment, the sensitivity can be determined by adapting interstitial, tissue, or cellular glucose levels for a fixed dose to blood glucose level. Other determinations of insulin sensitivity are possible, including clinically-derived values.
Based on the personal medication sensitivity, a medication sensitivity coefficient or coefficients can be found by proportioning the personal sensitivity to the population-based activity curve, if available (operation 153). The coefficient can be determined through area estimation, as further described below for dosed insulin with reference to FIGURE 15. Finally, once determined, the coefficient can be applied to the population-based activity curve to generate a personal activity model for the particular antidiabetic or oral medication (operation 154).

Calibrating an Oral Antidiabetic Medication Activity Curve

Changes to diabetes management are expected for Type 2 diabetics. However, dietary habits, personal well being, and other factors affecting both Type 1 and Type 2 diabetics can require adjustment to oral antidiabetic medication activity curves. FIGURE 14 is a process flow diagram showing a routine 160 for calibrating an oral antidiabetic medication activity curve for use with the method 120 of FIGURE 10. Calibration uses self-testing data to corroborate and refine the management tool.
Calibration can be performed regularly, or only as needed, based on factors relating to insulin, oral antidiabetic medication, and lifestyle, as further described below with reference to FIGURE 19. During each calibration, external feedback regarding the medication is aggregated into the management tool (operation 161) and applied to re-evaluate the medication sensitivity coefficient (operation 162). In addition, the feedback can be aggregated over several sample sets, or types of feedback, as further described below with reference to FIGURE 20. In a further embodiment, a threshold is applied to the feedback to prevent oscillations in changes to the activity curve due to minor and insignificant fluctuations. A revised personal antidiabetic or oral medication activity model can then be generated (operation 163) as a reflection of current circumstances.

Personalizing a Population-Based Insulin Activity Curve

Insulin is a peptide hormone composed of amino acid residues. Conventional insulin preparations are human insulin analogs that provide therapeutic effect along a projected activity curve that is characterized by time of onset, peak time, and duration of action. FIGURE 15 is a graph 170 showing, by way of example, an insulin activity curve 171 for lispro, an insulin analog manufactured by Eli Lilly and Company, Indianapolis, IN, and marketed under the Humalog brand name. The x-axis represents time in minutes and the y-axis represents the rate of glucose infusion measured in milligrams per minute per kilogram (mg/min/kg). Insulin activity curves are widely available for other insulin preparation types, and form the same general profile varied by time of onset, peak time, and duration.

The insulin activity curves published by insulin manufacturers and other authoritative sources are generally constructed as glucose clamp curves from normal volunteers, rather than diabetics, so resultant insulin activity curves must be estimated from the published curves. Insulin activity can be modeled for a diabetic patient by first estimating insulin sensitivity for an insulin preparation type. The insulin sensitivity refers to the overall change in blood glucose for a given dose of insulin, which the equivalent of integrating the area A under the insulin activity curve 171 and proportioning the area A to the net change in blood glucose 172. Thus, insulin sensitivity s can be estimated by taking the first order derivative of the rate of change of blood glucose over time:

$$s = \int \frac{dx}{dt} \tag{1}$$

where x is glucose infusion rate and t is time. Other estimates of insulin sensitivity are possible.

The insulin sensitivity is then proportioned to the population-based insulin activity curve 171 using an insulin sensitivity coefficient k for the patient. For example, if a 1.0 unit dose caused a 30 mg/dL drop in blood glucose, the area A would equal 30, and the magnitude of the values of each point along the x-axis are adjusted to the ratio of the insulin sensitivity coefficient k to the population-based value to yield a bioactivity curve for a 1.0 unit dose. Other applications of insulin sensitivity coefficients are possible.

Personal Insulin Activity Curve

The insulin sensitivity coefficient can be applied to the population-based insulin activity curve to generate a personal insulin activity model for the patient. FIGURE 16 is a diagram showing, by way of example, a screen shot 180 of a personal insulin activity curve for display in the graphical user interface 80 of FIGURE 6. The x-axis 181 again represents time in minutes and the y-axis 182 represents incremental blood glucose decrease measured in mg/dL.

The personal insulin activity model can be depicted through an approximation, plotted as a patient-specific insulin activity curve 183, which mimics the shape of the population-based insulin activity curve by a curvilinear ramp 184 to the peak activity time, followed by an exponential decay τ 126. A first modeling coefficient is used for the time to peak activity, called the filter length. A second coefficient is used for overall duration or decay of activity τ. The insulin sensitivity coefficient is applied to the population-based insulin activity curve through the filter length and τ. Thus, for a patient insulin sensitivity coefficient of 90%, for example, the patient-specific insulin activity curve 183 reflects a ten percent decrease in insulin sensitivity over corresponding population-based results. Other forms of and coefficients for models of population-based insulin activity curves are possible.

In a further embodiment, a time factor adjustment can be applied to get an overall insulin activity curve appropriately adjusted for an individual diabetic. For example, if the insulin activity curve for the individual was shorter in duration, the population-based insulin activity curve would be proportionally decreased along the time axis. Other personal insulin activity models are possible.

Digestive Response Modeling

Digestive speed and amplitude are initially modeled through ingestion of a standardized test meal from which a digestive response curve can be established and calibrated. The digestive response curve is thus adapted to the patient by factoring individual sensitivities into the personal predictive management tool.

In a manner similar to insulin activity curve determination, digestive response curve establishment and calibration provides a model from which other food responses can be projected. FIGURE 17 is a process flow diagram showing a routine 190 for establishing a digestive response curve for use with the method 120 of FIGURE 10. Determining a digestive response curve is an empirical process performed by the patient prior to commencing automated diabetes management and repeated as necessary to calibrate or fine-tune the curve.

Initially, the patient must undertake a fast (operation 191), preferably overnight and limited to only clear liquids or water. Following fasting, an initial test for blood glucose level is made (operation 192) to establish a starting point for blood glucose rise. The patient thereafter consumes a standardized and timed test meal (operation 193), such as a specific number of oat wafers, manufactured, for instance, by Ceapro Inc., Edmonton, Canada, or similar calibrated consumable. The test meal contains a known quantity of carbohydrate. A second test for postprandial blood glucose is made after a set time period (operation 194). If desired, further post-meal blood glucose tests can be performed (not shown), although standardized test meals are designed to exhibit peak blood glucose rise after a fixed time period and further testing generally yields nominal additional information. Finally, a carbohydrate sensitivity coefficient established by plotting the observed baseline and peak blood glucose levels on a personal digestive response curve (operation 195). The protocol can be repeated, as needed, to ascertain and resolve any variability in testing results. In a further embodiment, population-based digestive response curves can be used in lieu of or in combination with an empirically-determined personal digestive response curve.

Personal Digestive Response Curve

A digestive response curve estimates digestive speed and amplitude for an individual patient, which traces blood glucose rise, peak, and fall following food consumption. FIGURE 18 is a graph 200 showing, by way of example, a personal digestive response curve 203 for a standardized meal. The x-axis 201 represents time in minutes and the y-axis 202 represents a cumulative rise of blood glucose measured in milligrams per deciliter (mg/dL). The amplitude of the curve 203 is patient-dependent, as is the timing of the rise. However, where the carbohydrate content of the standardized test meal is precisely known, the curve 203 can be adapted to other types of foods to estimate glycemic effect and counteraction by insulin dosing, such as described in commonly-assigned U.S. Patent application, entitled "System And Method For Actively Managing Type 1 Diabetes Mellitus On A Personalized Basis," Serial No. 12/030,087, filed February 12, 2008, pending; U.S. Patent application, entitled "System and Method for Managing Type 1 Diabetes Mellitus Through a Personal Predictive Management Tool," Serial No. 12/030,120, filed February 12, 2008, pending, U.S. Patent application, entitled "System And Method For Actively Managing Type 2 Diabetes Mellitus On A Personalized Basis," Serial No. 12/030,097, filed February 12, 2008, pending; U.S. Patent application, entitled "System and Method for Managing Type 2 Diabetes Mellitus Through a Personal Predictive Management Tool," Serial No. 12/030,130, filed February 12, 2008, pending, the disclosures of which are incorporated by reference.

Characteristics Affecting Management of Type 1 and Type 2 Diabetics

A range of characteristics affect the effectiveness of the automated diabetes management tool. FIGURE 19 is a process flow diagram showing, by way of example, characteristics 211 affecting diabetes management 210. Other characteristics can also affect diabetes management.

Type 1 diabetics are dependent on externally supplied insulin, as well as end-stage Type 2 diabetes with failed insulin production. The basic principle underlying insulin therapy is to use short-acting insulin to cover meals and longer-acting insulin between meals and overnight. Thus, insulin considerations 212 include the type of insulin preparation administered and the timing and doses of insulin. Additionally, insulin can be administered through subcutaneous injection, insulin pump, inhalation, and transdermal delivery. Other modes of insulin administration are possible. Other insulin considerations are possible.

Lifestyle considerations 213 factor heavily into determinations of insulin dosing. Food consumption considerations 214 include the types, amounts, and combinations of foods consumed, particularly in terms of carbohydrate content and glycemic index. Food includes beverages that will lead to an eventual rise in blood glucose, such as high sucrose drinks, like orange juice. In addition, personal food preferences, familial traditions, preferred seasonings and accompaniments, ethnicity, social eating patterns, and similar factors can also indirectly influence blood glucose. Exercise considerations 215 includes any form of physical exertion or activity likely to require a measurable caloric outlay. Finally, patient condition 216 can cause blood glucose to abnormally rise or fall, depending upon the condition. For instance, a virus, such as influenza, can cause blood glucose to decrease, while emotional stress can raise blood glucose through stimulation of adrenaline. The normal pancreas in non-diabetic individuals manages all of these shifts in glucose metabolism smoothly to prevent both too high and too low values of glucose. Other lifestyle considerations are possible.

Type 2 diabetics also suffer some combination of defective insulin secretion, insulin resistance, and reduced insulin sensitivity 217. The level of affect tends to change over time as the disease progresses, although, at least in the early stage, positive changes to exercise, diet, and weight loss may temporarily reverse insulin resistance. Other insulin resistance considerations are possible.

Factors Bearing on Type 1 and Type 2 Diabetics Management

Diabetes management is a dynamic process that must evolve with patient condition to remain effective, especially for Type 2 diabetics. FIGURE 20 is a process flow diagram showing, by way of example, factors 221 bearing on personal predictive diabetes management 220. Each factor, when known, constitutes feedback that can potentially have an affect on the efficacy and accuracy of an underlying diabetes management tool.

Blood glucose testing results provide strong corroboration of the management tool's accuracy. Test results can be provided through empirical measures 222 from self-testing, which can be compared to expected blood glucose levels as predicted by the management tool. Similarly, clinical monitoring data 223, such as glycated hemoglobin, fructosamine, urinary glucose, urinary ketone, and interstitial, tissue, or cellular glucose testing results, can be used. Other forms of blood glucose testing results are possible.

Other factors may also apply. For instance, event data 224 details specifics, such as insulin basal dose, insulin bolus dose, insulin bolus timing, insulin resistance level, period of day, time of day, medications, patient activity level, and patient physical condition. Other forms of event data are possible. Insulin preparation type 225 should seldom vary, but when affected, can signal the need to re-evaluate and calibrate the management tool. Finally, selecting a population-based insulin activity curve for a patient population most appropriately corresponding to the quantitative characteristics 226 of the patient can improve the type of personal insulin activity model generated and subsequently refined to match the specific needs of the patient. Finally, the types and dosing of oral antidiabetic medications 227 for Type 2 and select Type 1 diabetics can directly or indirectly affect blood glucose. Moreover, the medications taken by a Type 2 diabetic will likely change as the disease progresses. Other factors bearing on diabetes management are possible.

System

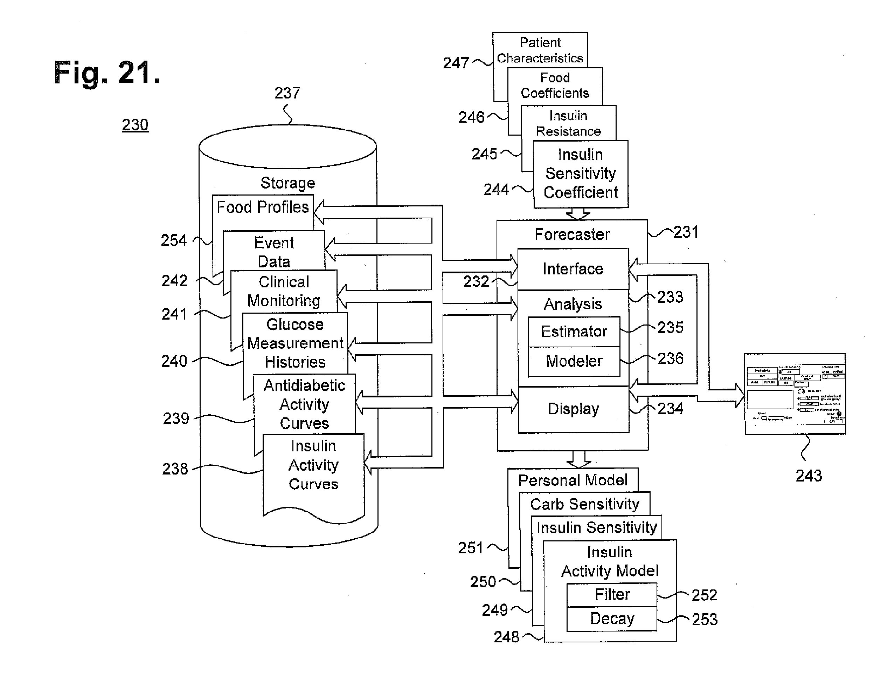

Automated diabetes management can be provided on a system implemented through a patient-operable device, as described above with reference to FIGURE 5. FIGURE 21 is a block diagram showing for a system 230 for establishing a personalized diabetes management tool for Type 1 and Type 2 diabetes mellitus, in accordance with one embodiment. The patient-operable device must accommodate user inputs, provide a display capability, and include local storage and external interfacing means.

In one embodiment, the system 230 is implemented as a forecaster application 231 that includes interface 232, analysis 233 and display 234 modules, plus a storage device 237. The storage device 237 is used to maintain a database or other form of structured data store in which glucose measurements, physiological data, and other aspects of patient medical histories are kept. Other modules and devices are possible.

The interface module 232 accepts user inputs, such as insulin sensitivity coefficient 244, insulin resistance 245 (Type 2 only), food coefficients 246, and patient-specific characteristics 247. Other inputs, both user-originated and from other sources, are possible. In addition, in a further embodiment, the interface module 232 facilitates direct interconnection with external devices, such as a blood, interstitial, tissue, or cellular glucose monitor, or centralized server (not shown). The interface module 232 can also provide wired or wireless access for communication over a private or public data network, such as the Internet. Other types of interface functionality are possible.

The analysis module 233 includes estimator 235 and modeler 236 submodules. The estimator submodule 235 determines an insulin sensitivity 249 by taking a derivative of the rate of change of blood glucose over time of a population-based insulin activity curve 238 maintained on the storage device 237. The modeler submodule 236 forms an insulin activity model 248 of the population-based insulin activity curve 238 by determining a filter length 252 and exponential decay 253. The modeler submodule 236 also forms activity curves for oral antidiabetic medications 239, as applicable, and a carbohydrate sensitivity 250 that includes a personal digestive response curve 203 (shown in FIGURE 18) determined, for instance, from a patient-supplied carbohydrate sensitivity coefficient 246 or through empirical testing with a standardized test meal. The insulin sensitivity coefficient 242 is applied to the insulin activity model 248 to form a patient-specific insulin response curve 183 (shown in FIGURE 16), which is combined with the oral antidiabetic medication activity curves 239, if applicable, and personal digestive response curve 202 to build a personalized diabetes management tool 251.

In a further embodiment, population-based digestive response curves 238, glucose measurement histories 240, clinical monitoring 241, food profiles 254, and event data 242, as well as other external forms of data, are also maintained on the storage device 237. This information is used to re-evaluate the insulin sensitivity coefficient 244 and to calibrate the personal insulin activity model 248. Other types of analysis functionality are possible.

Finally, the display module 234 generates a graphical user interface 243, through which the user can interact with the forecaster 231. The user interface 243 and its functionality are described above with reference to FIGURE 6.

HbA1c Estimation and Accuracy

HbA1c is formed when glucose irreversibly binds to hemoglobin in red blood cells to form a stable glycated hemoglobin complex. Physicians routinely check the HbA1c percentile of their diabetic patients about every three to six months to gauge their patients' control over their blood glucose. An HbA1c measurement represents the percentile of hemoglobin glycated over the life span of a patient's red blood cells, which is about 120 days. An HbA1c measurement of under seven percent reflects good patient blood glucose control.

HbA1c is a long term and stable metric that is directly proportional to the concentration of glucose in the blood stream or body tissues, particularly interstitially. In contrast to HbA1c, daily blood glucose measurements are short term metrics, which are subject to wide variation. Typically, a patient performs self-testing of blood glucose several times each day. Ideally, a diabetic's blood glucose is maintained between 70 mg/dL and 120 mg/dL between meals and under 140 mg/dL at two hours postprandial. A patient's blood glucose profile will therefore vary throughout each day, even when good control over blood glucose is exercised. Unlike blood glucose, HbA1c is a relatively stable physiometric value that is not subject to the fluctuations ordinarily experienced with daily blood glucose levels and the percentile of hemoglobin glycated remains fairly stable when viewed over a short time period.

Stability makes HbA1c an effective tool for managing glucose when provided in combination with daily self-testing. FIGURE 22 is a graph 260 showing, by way of example, mean blood glucose profile 261 compared to HbA1c level 262 over a set time period. The x-axis represents time in days. The left-hand y-axis represents mean daily blood glucose level in mg/dL. The right-hand y-axis represents percentile of glycation of hemoglobin. The mean blood glucose profile 261 follows what a diabetic patient with an abrupt change in blood glucose control would experience, such as a Type 2 diabetic who has stopped taking his antidiabetic medications or insulin. Although mean blood glucose steeply rises over a short time period, HbA1c responds by rising at a much slower rate over a significantly longer time period. Taken alone, the mean blood glucose profile 261 only shows a limited and potentially misleading part of the overall picture. At thirty days, the daily mean blood glucose level is about 150 mg/dL, which, while slightly elevated, is still close to an acceptable level. However, the HbA1c level 262 helps highlight the underlying and much more serious concern of higher hemoglobin glycation, as the percentile at thirty days exceeds eight percent, an uncomfortably and unhealthily high level that warrants prompt medical attention.

Graphical User Interface

Nevertheless, the relationship between HbA1c and daily glucose measurements, especially blood glucose measurements, may not be apparent to the average patient, particularly as HbA1c is generally only determined through laboratory analysis in connection with infrequent visits to a physician. For example, an illness, which results in abnormally high blood glucose in the week or two preceding an in-laboratory HbA1c assessment, may suggest overall poorer chronic blood glucose control than actually exists. Providing a diabetic with an estimate of their HbA1c on an on-going basis would help temper potential over-reliance on the occasional HbA1c test as the sole determinant of blood glucose control. FIGURE 23 is a diagram showing, by way of example, a screen shot of a graphical user interface 270 for providing an estimate of HbA1c 271 for use in the graphical user interface 80 of FIGURE 6. The estimate 271 is provided as a percentile of glycated hemoglobin. Additionally, an estimate of the accuracy 272 of the HbA1c estimate 271 is provided to reflect the level of confidence to be ascribed to the estimate provided. "Good" accuracy reflects high confidence in the HbA1c estimate 271, whereas "Poor" accuracy cautions against complete reliance on the estimate 271.

When used in combination with a personalized diabetes management tool, such as described *supra* with reference to FIGURE 6, the HbA1c estimate 271 and accuracy estimate 272 provide a longer-term estimation of personal glucose control than available through daily self-testing alone. Individual blood glucose readings 84 are entered throughout the day and the effects of diet, physical activity, insulin, if applicable, and other factors are reflected in a blood glucose forecast curve 87. However, the forecast curve 87 is short term and focused over a narrow time window that is affected by variables that can be generally be readily changed with immediate effect on the forecast curve 87. For instance, the patient could decide to eat less carbohydrate-rich foods to avoid a predicted spike in his blood glucose. In contrast, HbA1c is relatively unaffected by non-trending changes. In particular, day-to-day changes to individual medication, dietary selection, or physical activity may have little affect on HbA1c. Consequently, the HbA1c estimate 271 and accompanying accuracy estimate 272 enable the patient to see how he is tracking in personal glucose management relatively independent of the day-to-day variations in dietary, physical, and medical regimen that influence short term glucose levels.

Method

HbA1c can be estimated and made available to a patient by analysis of available glucose levels, including blood, interstitial, tissue, cellular and other glucose measures. FIGURE 24 is a process flow diagram a method 280 for providing a personalized tool for estimating glycated hemoglobin in accordance with a further embodiment. The method is performed as a series of process steps or operations and can be provided by the automated diabetes management tool, as described *supra*, or through a separate utility operating on a personal digital assistant or similar programmable device.

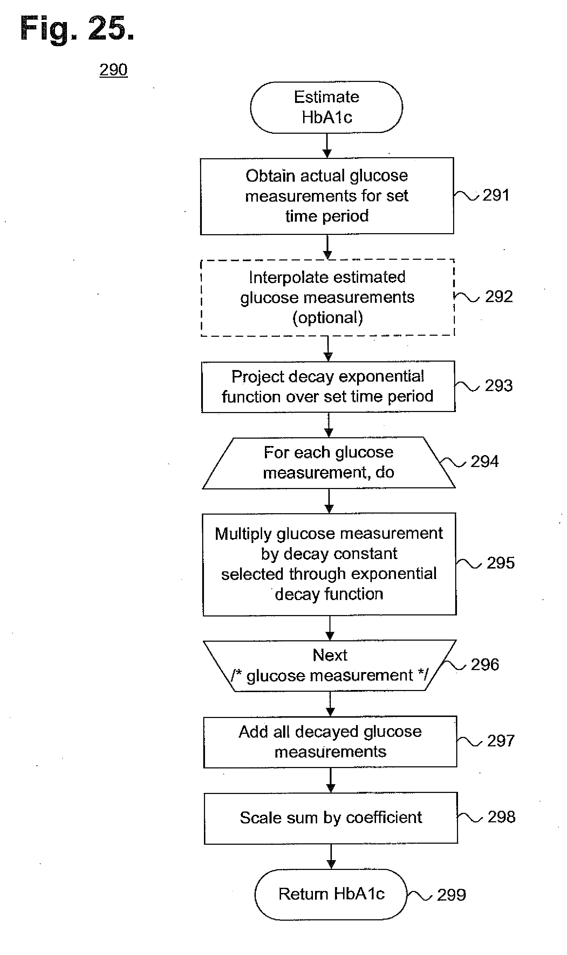

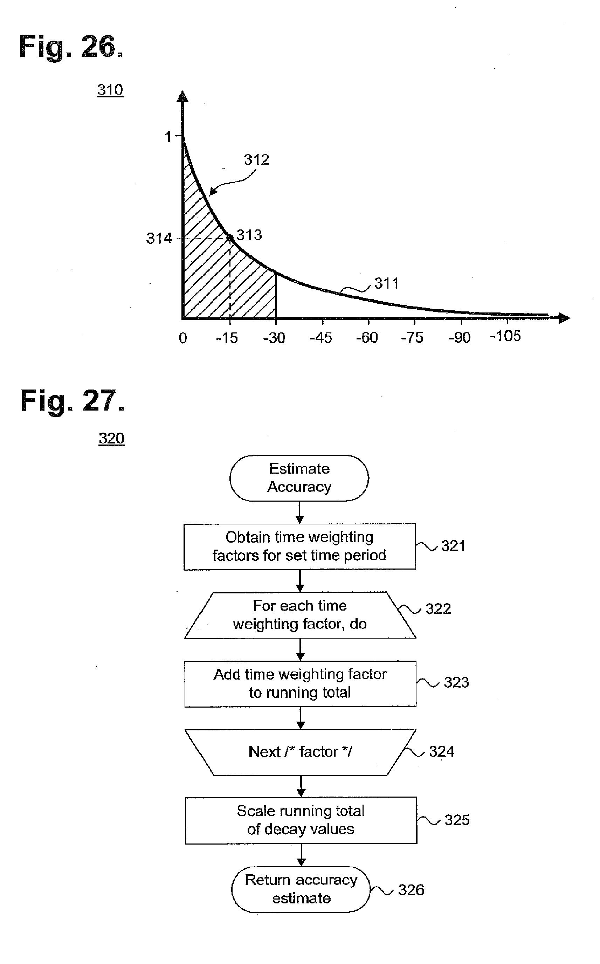

The estimates of HbA1c 271 and its ascribed accuracy 272 are both determined by evaluating glucose measurements through time-weighted analysis. HbA1c is estimated (operation 281) by applying an exponential decay function to time-averaged glucose measurements over a set time period, as further described below with reference to FIGURE 25. Accuracy is estimated (operation 282) by quantifying the quality of available glucose measurements, as further described below with reference to FIGURE 27. The resulting estimates of HbA1c 271 and ascribed accuracy 272 serve to inform a patient on how their personal glucose management efforts are progressing, especially during the interim between medical checkups.

Estimation of HbA1c

Each estimate of HbA1c 271 is based on the value and the timing of daily glucose measurements. FIGURE 25 is a flow diagram showing a routine 290 for estimating HbA1c for use with the method 280 of FIGURE 24. Clinically, an HbA1c percentile is related to the average blood glucose level taken over the past one to three months and is heavily weighted to favor the most recent two to four weeks. Each HbA1c estimate 271 is based on glucose measurements from a recent time period, generally over the most recent 90 to 120 days. Actual glucose measurements are obtained for each interval during the time period (block 291). The glucose measurements may be electronically stored as data values maintained in a database or memory.

The number of actual glucose level measurements available will depend upon the type of testing and the frequency of and consistency by which the patient performs self-testing. For instance, a continuous interstitial, tissue, or cellular glucose monitor may generate and store a new glucose reading once each minute or at any other regular interval. Alternatively, manual blood glucose self-testing using test strips and a blood glucose meter may yield as few as only one glucose measurement per day, or perhaps six readings, if blood glucose is measured between meals and postprandial. An estimate of HbAc can be formed based just on the glucose measurements actually available. Alternatively, in a further embodiment, additional glucose measurements can be estimated to fill in any gaps in actual glucose measurements. Such gaps can be filled by optionally interpolating estimated blood glucose levels (step 292) between adjacent pairs of actual glucose measurements. Preferably, the interpolation is taken at regular intervals, for instance, by the minute, by the hour, by the day, or by the week. The estimated glucose measurements can be determined by linear interpolation, such as a mean or average glucose value, or through exponential interpolation. Other glucose level estimations are possible.

Together, the actual and estimated glucose levels, when generated, form a data set that extends over the entire time period. An exponential decay function is then projected over the time period (block 293). The sensitivity of HbA1c decays at a rate equivalent to the integral of the most recent 30 days of blood glucose measurements, which can be approximated as fifty percent of the total area under a decay curve. This relationship can be modeled as an exponential decay function $f(x)$ expressed as:

$$f(x) = e^{-\lambda} \tag{2}$$

where $x$ is a regular interval within the time period, such as a minute, hour, day, or week, and $\lambda$ is a decay constant generally occurring between 40 and 60 percent, frequently 50 percent. Other decay functions and rates of decay are possible.

Referring to FIGURE 26, a graph 310 showing, by way of example, an exponential decay function projected over a set time period is provided. The $x$-axis represents time in order of increasing age in days. The $y$-axis represents ordinal numbers. The exponential decay function defines a continuous decay curve 311 asymptotically diminishing towards zero. Empirically, HbA1c has been observed to reflect fifty percent of the value of glucose levels measured over the most recent 30 days and particularly over the most recent 2 weeks, which corresponds to 50% of the area 312 under the decay curve 311, as indicated by shaded lines. A time weighting factor or value for a decay constant 314 can be derived by the intersection 313 of a regular time interval and the decay curve. Referring back to FIGURE 25, each glucose measurement is evaluated iteratively (blocks 294-296) to multiply the glucose measurement at each point throughout the set time period by a corresponding decay constant selected through the exponential decay function (block 295). Thereafter, all actual glucose measurements and estimated glucose measurements, when available, as adjusted by their decay constants, are added (block 297) and the sum of the glucose measurements are scaled by a coefficient (block 298) to yield an HbA1c estimate 271 (block 299). The sum of the decayed glucose measurements are scaled by a constant coefficient, such as 5/100 for blood glucose, which empirically yields a scale factor appropriate to reflect HbA1c. Other scaling coefficients may apply for interstitial, tissue, or cellular glucose or other measures.

Ascription of Accuracy

As the value of HbA1c provided is only an estimate, an estimate of its accuracy 272 is also provided to indicate the level of confidence that a patient should ascribe to the estimate. FIGURE 27 is a flow diagram showing a routine 320 for estimating accuracy for use with the method 280 of FIGURE 24. The ascription of accuracy reflects that a higher number of glucose measurements more recently obtained leads to better estimation accuracy.

The accuracy is determined by summing only the actual time weighting factors at the time of the glucose measurements. The time weighting factors are obtained for each glucose sample data point during the time period (block 321). Each time weighting factor is evaluated iteratively (blocks 322-324) by adding the factor to a running total of all the time weighting factors (block 323). Thereafter, the running total is scaled (block 325) to yield an estimate of the accuracy 272 of the corresponding HbA1c estimate 271 (block 326). The accuracy estimate can then be displayed along a gradient that indicates the relative goodness of the HbA1c estimate.

Scaling can be provided by determining the percentage of intervals in the time period that have actual glucose measurements available and setting the estimate of the accuracy 272 to a value relative to the percentage determined. With a 30-day time period with one-minute intervals, even a diabetic patient that performs self-testing consistently before every meal and two hours after each meal will only have a sparsely populated set of actual glucose measurements, so the scaling needs to adjust the estimate of accuracy 272 based on the reality of real world self-testing where, for example, six readings of glucose in a day is considered "good." Other forms of scaling or normalization are possible.

System

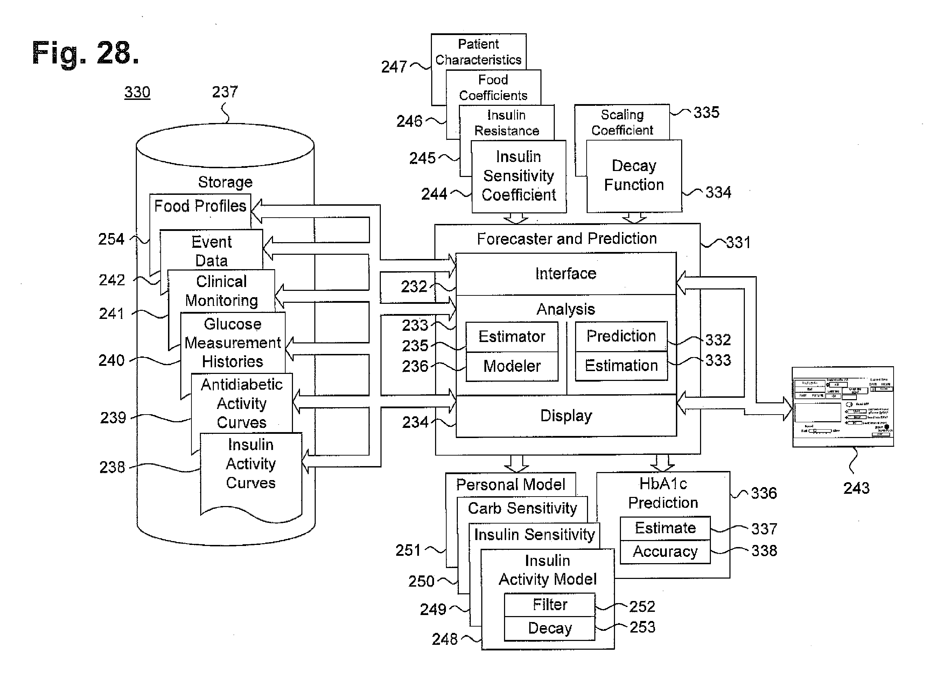

Estimates of HbA1c can be provided on the same system used for automated diabetes management. FIGURE 28 is a block diagram showing for a system 330 for providing a personalized tool for estimating glycated hemoglobin, in accordance with a further embodiment. The system includes a patient-operable device able to accommodate user inputs, provide a display capability, and include local storage and external interfacing means, as described above with reference to FIGURE 21.

In one embodiment, the system 330 is implemented as a forecaster and prediction application 331 that includes the interface 232, analysis 233 and display 234 modules, and storage device 237, as described *supra*, with additional functionality as follows. In addition to the estimator 235 and modeler 236 submodules, the analysis module 233 includes prediction 332 and estimation 333 submodules, which respectively generate an HbA1c prediction 336 that includes an estimate of HbA1c 337 and an estimate of the accuracy 338 of the HbA1c estimate 337. The prediction module 332 estimates glucose measurements to fill in gaps in the glucose measurement histories 240, and generates the HbA1c estimate 337 by applying a decay function 334 and scaling coefficient 335 to the actual and estimated glucose measurements. The estimation module 333 takes a summation of the actual glucose measurements and ascribes a relative degree of accuracy to the estimate by normalizing the summation. Other types of analysis functionality are possible.

While the invention has been particularly shown and described as referenced to the embodiments thereof, those skilled in the art will understand that the foregoing and other changes in form and detail may be made therein without departing from the spirit and scope.

**Claims**

1. A system (230) for creating a personalized tool predicting a time course (87) of blood glucose affect in diabetes mellitus, comprising:

    stored information (237), comprising:

a substance (81, 82, 83) whose introduction into a diabetic patient (21) triggers a physiological effect relative to the diabetic patient's blood glucose (84); and

a time course (87) and an amplitude of change (87) over which the physiological effect is expected to occur;

an analysis module (233) configured to determine a factor (221) specific to the physiological effect on the diabetic patient (21) and to adjust the time course (87) and the amplitude of change (87) in relation to the diabetic patient-specific factor (221); and

a modeler module (236) configured to model (248) the physiological effect on a curve (203) with the time course (87) and the amplitude of change (87) mapped as a function of a quantity of the substance (81, 82, 83).

2. A system (230) according to Claim 1, wherein a dosed insulin preparation is specified as the substance (81, 82, 83) and an insulin sensitivity (92) is specified as the factor (221), such that the affect of the substance (81, 82, 83) comprises mediating transport of blood glucose (84) into cells in proportion to the insulin sensitivity (92).

3. A system (230) according to Claim 2, wherein the stored information further comprises an insulin activity curve (203) for the dosed insulin preparation comprising a time course (87) and an amplitude of change (87) applicable to a patient population, the system (230) further comprising:

an evaluation module (235) configured to identify the insulin sensitivity (92) by taking a derivative of the rate of change of blood glucose (84) over time for the dosed insulin preparation, wherein the insulin sensitivity (92) is applied to the patient population insulin activity curve (203) over a duration of action of the dosed insulin preparation.

4. A system (230) according to Claim 2, wherein the diabetic patient (21) comprises a Type 1 diabetic and the dosed insulin preparation is required for inclusion in the stored information.

5. A system (230) according to Claim 1, wherein one of an antidiabetic medication and an oral medication is specified as the substance (81, 82, 83) and a physiological reaction of the patient's body to the medication is specified as the factor (221), such that the affect of the substance (81, 82, 83) comprises triggering a hematological interaction with blood glucose (84) as the physiological reaction.

6. A system (230) according to Claim 1, wherein carbohydrates are specified as the substance (81, 82, 83) and a carbohydrate sensitivity is specified as the factor (221), such that the affect of the substance (81, 82, 83) comprises causing a rise in blood glucose (84) in proportion to the carbohydrate sensitivity.

7. A system (230) according to Claim 1, wherein the diabetic patient (21) comprises a Type 2 diabetic, further comprising:

during an early stage of Type 2 diabetes, wherein the substance (81, 82, 83) is omitted from the stored information;

during a middle stage of the Type 2 diabetes, wherein one of an antidiabetic medication and an oral medication is further specified as the substance (81, 82, 83) and a physiological reaction of the patient's body to the medication as the factor (221); and

during an end-stage of the Type 2 diabetes, wherein a dosed insulin preparation is further specified as the substance (81, 82, 83) and an insulin sensitivity (92) as the factor (221).

8. A method (70) for creating a personalized tool predicting a time course (87) of blood glucose affect in diabetes mellitus, comprising:

selecting a substance (81, 82, 83) whose introduction into a diabetic patient (21) triggers a physiological effect relative to the diabetic patient's blood glucose (84);

determining a time course (87) and an amplitude of change (87) over which the physiological effect is expected to occur;

adjusting the time course (87) and the amplitude of change (87) in relation to a factor (221) specific to the physiological effect on the diabetic patient (21); and

modeling the physiological effect on a curve (203) with the time course (87) and the amplitude of change (87) mapped as a function of a quantity of the substance (81, 82, 83).

9. A method (70) according to Claim 8, further comprising:

specifying a dosed insulin preparation as the substance (81, 82, 83) and an insulin sensitivity (92) as the factor (221), wherein the affect of the substance (81, 82, 83) comprises mediating transport of blood glucose (84) into cells in proportion to the insulin sensitivity (92).

**10.** A method (70) according to Claim 9, further comprising:

identifying an insulin activity curve (203) for the dosed insulin preparation comprising a time course (87) and an amplitude of change (87) applicable to a patient population;
determining the insulin sensitivity (92) by taking a derivative of the rate of change of blood glucose (84) over time for the dosed insulin preparation; and
applying the insulin sensitivity (92) to the patient population insulin activity curve (203) over a duration of action of the dosed insulin preparation.

**11.** A method (70) according to Claim 9, wherein the diabetic patient (21) comprises a Type 1 diabetic, further comprising:

requiring inclusion of the dosed insulin preparation.

**12.** A method (70) according to Claim 8, further comprising:

specifying one of an antidiabetic medication and an oral medication as the substance (81, 82, 83) and a physiological reaction of the patient's body to the medication as the factor (221), wherein the affect of the substance (81, 82, 83) comprises triggering a hematological interaction with blood glucose (84) as the physiological reaction.

**13.** A method (70) according to Claim 8, further comprising:

specifying carbohydrates as the substance (81, 82, 83) and a carbohydrate sensitivity as the factor (221), wherein the affect of the substance (81, 82, 83) comprises causing a rise in blood glucose (84) in proportion to the carbohydrate sensitivity.

**14.** A method (70) according to Claim 8, wherein the diabetic patient (21) comprises a Type 2 diabetic, further comprising:

during an early stage of Type 2 diabetes, omitting the substance (81, 82, 83);
during a middle stage of the Type 2 diabetes, further specifying one of an antidiabetic medication and an oral medication as the substance (81, 82, 83) and a physiological reaction of the patient's body to the medication as the factor (221); and
during an end-stage of the Type 2 diabetes, further specifying a dosed insulin preparation as the substance (81, 82, 83) and an insulin sensitivity (92) as the factor (221).

Fig. 1 (prior art).

Fig. 2.

Fig. 3B. (prior art)

36

31

37

38

Fig. 3A. (Prior Art)

30

32

31

33

35

34

Fig. 4A.

50

Fig. 3C (prior art).

39

# Fig. 4B.

<u>57</u>

51

58

59

60

# Fig. 4C.

<u>61</u>

51

62

64

63

67

65

66

EP 2 223 648 A1

24

# Fig. 5.

70

Plan
(71)

Calibrate
(73)

Model
(72)

# Fig. 6.

80

| File | Edit | View | | | | □ ▢ ☒ |

Blood Glucose (mg/dL) vs Time (hours)

87

| What If | EDIT | BG | ~84 |
| FOOD | Insulin | Medications | ~82 |

86

BG Est: 203    Ins Deficit: 0

STOP

83    85    81

# Fig. 7.

90

| □ Insulin Bolus | [X] |
|---|---|

**Insulin Type**

Humalog
Novolog
Ultralente
Lantus
Lente
Regular
NPH
70/30

} 91

APPLY ~ 94

92 ~ ▲☺ [ 4.0 ] **Insulin Sensitivity**

93 ~ ▲☺ [ 30.0 ] **Insulin bolus bump (U)**

# Fig. 8.

100

| □ | [X] |
|---|---|

**Medication Type**

Exenatide (Byetta)
Pramlintide (Symlin)
Sulfonylurea (Glucotrol)
Meglinitinide
Nateglinitide
Biguanides
Thiazolidinedione (Actos)
Alpha-glucose inhibitor
Metformin (Glucophage)

} 101

APPLY

102

# Fig. 9.

110

| □ Meal | [X] |
|---|---|

Waffles (two whole)
Club sandwich (half)
Granola bar
Hamburger
Cheeseburger
Orange juice (6 oz)
Soft drink, non-diet (12 oz)
Sirloin steak (16 oz)
Fried chicken (drumstick)
Mashed potatoes
Spaghetti (al dente)
Spaghetti sauce, tomato
Meatballs (two hamburger)
Peas, frozen (4 oz)
Corn, canned (4 oz)
Cheesecake (one slice)
Ice cream, chocolate
Hard candy (one piece)

111

115

112 — Add Item

**Glycemic Index** [ 5 ▼ ] 113

**Carbohydrates** [ 30 ▼ ] 114

Finished? 116

# Fig. 10.

120

Establish management model (121) → Calibrate management model (122)

# Fig. 11.

130

Identify appropriate population-based insulin activity curve (131)

Determine personal insulin sensitivity for patient (132)

Proportioning to determine coefficient (133)

Generate personal insulin activity model (134)

# Fig. 12.

140

Aggregate external feedback (141)

Reevaluate insulin sensitivity coefficient (142)

Reapply Insulin sensitivity coefficient (143)

# Fig. 13.

150

# Fig. 14.

160

# Fig. 15.

# Fig. 16.

180

# Fig. 17.

190

Fast
(191)

Generate
personal digestive
response curve
(195)

Initial Testing
(192)

Post Prandial
Testing
(194)

Test Meal
Consumption
(193)

# Fig. 18.

200

202

150 —

120 —

90 —

60 —

30 —

0 —

Cumulative Blood Glucose
Change (mg/dL)

203

201

100    200    300    400    500

Time After Ingestion (min)

# Fig. 19.

210

# Fig. 20.

220

Fig. 21.

# Fig. 22.

260

# Fig. 23.

270

# Fig. 24.

280

# Fig. 25.

290

```
        ┌─────────────────┐
        │    Estimate     │
        │     HbA1c       │
        └─────────────────┘
                 │
                 ▼
  ┌───────────────────────────┐
  │   Obtain actual glucose   │
  │   measurements for set    │ ⌐⌐ 291
  │       time period         │
  └───────────────────────────┘
                 │
                 ▼
  ┌ ─ ─ ─ ─ ─ ─ ─ ─ ─ ─ ─ ─ ┐
  │  Interpolate estimated    │
  │  glucose measurements     │ ⌐⌐ 292
  │       (optional)          │
  └ ─ ─ ─ ─ ─ ─ ─ ─ ─ ─ ─ ─ ┘
                 │
                 ▼
  ┌───────────────────────────┐
  │  Project decay exponential │ ⌐⌐ 293
  │  function over set time period │
  └───────────────────────────┘
                 │
                 ▼
   ╱─────────────────────────╲
  ╱    For each glucose        ╲ ⌐⌐ 294
  ╲    measurement, do         ╱
   ╲─────────────────────────╱
                 │
                 ▼
  ┌───────────────────────────┐
  │ Multiply glucose measurement │
  │      by decay constant      │ ⌐⌐ 295
  │  selected through exponential │
  │       decay function        │
  └───────────────────────────┘
                 │
                 ▼
   ╲─────────────────────────╱
    ╲        Next            ╱ ⌐⌐ 296
     ╲ /* glucose measurement */
      ╲─────────────────────╱
                 │
                 ▼
  ┌───────────────────────────┐
  │   Add all decayed glucose   │ ⌐⌐ 297
  │       measurements          │
  └───────────────────────────┘
                 │
                 ▼
  ┌───────────────────────────┐
  │   Scale sum by coefficient  │ ⌐⌐ 298
  └───────────────────────────┘
                 │
                 ▼
        ┌─────────────────┐
        │   Return HbA1c  │ ⌐⌐ 299
        └─────────────────┘
```

# Fig. 26.

310

# Fig. 27.

320

# Fig. 28.

EP 2 223 648 A1

Europäisches
Patentamt

European
Patent Office

Office européen
des brevets

**EUROPEAN SEARCH REPORT**

Application Number

EP 10 15 3553

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 822 715 A (WORTHINGTON DAVID R L [US] ET AL) 13 October 1998 (1998-10-13) * column 5, line 30 - column 11, line 11 * * column 16, line 23 - column 18, line 21 * * figures * | 1-14 | INV. A61B5/00 |
| X | US 2001/047252 A1 (BROWN STEPHEN J [US]) 29 November 2001 (2001-11-29) * paragraphs [0039] - [0070] * * figures 1-4 * | 1-14 | |
| X | EP 2 023 256 A1 (NOVO NORDISK AS [DK]) 11 February 2009 (2009-02-11) * paragraphs [0054] - [0060] * * paragraphs [0066] - [0091] * * figures * | 1,2,4-9, 11-14 | |
| X | EP 1 281 351 A2 (ROCHE DIAGNOSTICS GMBH [DE]; HOFFMANN LA ROCHE [CH]) 5 February 2003 (2003-02-05) * paragraphs [0031] - [0040] * * figures * | 1,2,4-9, 11-14 | TECHNICAL FIELDS SEARCHED (IPC) A61B G06F |

The present search report has been drawn up for all claims

| Place of search | Date of completion of the search | Examiner |
|---|---|---|
| The Hague | 21 May 2010 | Bataille, Frédéric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 10 15 3553

This annex lists the patent family members relating to the patent documents cited in the above-mentioned European search report.
The members are as contained in the European Patent Office EDP file on
The European Patent Office is in no way liable for these particulars which are merely given for the purpose of information.

21-05-2010

| Patent document cited in search report | | Publication date | Patent family member(s) | | Publication date |
|---|---|---|---|---|---|
| US 5822715 | A | 13-10-1998 | AU | 1309700 A | 17-04-2000 |
| | | | WO | 0018293 A1 | 06-04-2000 |
| | | | US | 6379301 B1 | 30-04-2002 |
| | | | US | 6167362 A | 26-12-2000 |
| | | | US | 6233539 B1 | 15-05-2001 |
| | | | US | 5956501 A | 21-09-1999 |
| US 2001047252 | A1 | 29-11-2001 | NONE | | |
| EP 2023256 | A1 | 11-02-2009 | CA | 2693122 A1 | 05-02-2009 |
| | | | EP | 2186032 A1 | 19-05-2010 |
| | | | WO | 2009016050 A1 | 05-02-2009 |
| EP 1281351 | A2 | 05-02-2003 | AT | 413654 T | 15-11-2008 |
| | | | CA | 2394900 A1 | 31-01-2003 |
| | | | ES | 2316506 T3 | 16-04-2009 |
| | | | JP | 4231253 B2 | 25-02-2009 |
| | | | JP | 2003079723 A | 18-03-2003 |
| | | | JP | 2006175227 A | 06-07-2006 |
| | | | US | 2003028089 A1 | 06-02-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6168563 B, Brown
- US 6024699 A, Surwit
- US 12030130 B
- US 12030087 B
- US 12030120 B
- US 12030097 B